(19) Europäisches Patentamt / European Patent Office / Office européen des brevets

(11) **EP 1 980 561 B1**

(12) **EUROPEAN PATENT SPECIFICATION**

(45) Date of publication and mention of the grant of the patent: **09.10.2013 Bulletin 2013/41**

(51) Int Cl.: ***C07D 471/04*** *(2006.01)* ***A61K 31/437*** *(2006.01)* ***A61P 35/00*** *(2006.01)*

(21) Application number: **08153430.7**

(22) Date of filing: **27.03.2008**

(54) **Substituted 1h-pyrazolo[3,4-b] pyridine derivatives active as kinase inhibitors**

Substituierte 1h-Pyrazolo[3,4-b]-pyridin-Derivate, die als Kinasehemmer aktiv sind

Dérivés de pyridines 1h-pyrazolo[3,4-b] substitués actifs en tant qu'inhibiteurs de kinase

(84) Designated Contracting States: **DE FR GB IT**

(30) Priority: **30.03.2007 EP 07105368**

(43) Date of publication of application: **15.10.2008 Bulletin 2008/42**

(73) Proprietor: **NERVIANO MEDICAL SCIENCES S.R.L.**
**20014 Nerviano (MI) (IT)**

(72) Inventors:
- **Piutti, Claudia**
  **20014, Nerviano (Milan) (IT)**
- **Banfi, Patrizia**
  **20015, Parabiago ((Milan) (IT)**
- **Mongelli, Nicola**
  **20137, Milan (IT)**
- **Veronesi, Marina**
  **20148, Milan (IT)**
- **Casuscelli, Francesco**
  **20015, Parabiago (MI) (IT)**

(56) References cited:
**WO-A-2005/011681** **WO-A-2006/063805**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

EP 1 980 561 B1

## Description

## BACKGROUND OF THE INVENTION

### Field of the invention

The present invention relates to certain substituted 1H- pyrazolo [3, 4- b] pyridine- 3- yl- acrylamide compounds, which modulate the activity of protein kinases. The compounds of this invention are therefore useful in treating diseases caused by dysregulated protein kinase activity. The present invention also provides methods for preparing these compounds, pharmaceutical compositions comprising these compounds, and methods of treating diseases utilizing pharmaceutical compositions comprising these compounds.

### Discussion of the Background

The malfunctioning of protein kinases (PKs) is the hallmark of numerous diseases. A large share of the oncogenes and proto-oncogenes involved in human cancers code for PKs. The enhanced activities of PKs are also implicated in many non-malignant diseases, such as benign prostate hyperplasia, familial adenomatosis, polyposis, neuro-fibromatosis, psoriasis, vascular smooth cell proliferation associated with atherosclerosis, pulmonary fibrosis, arthritis glomerulonephritis and post-surgical stenosis and restenosis.

PKs are also implicated in inflammatory conditions and in the multiplication of viruses and parasites. PKs may also play a major role in the pathogenesis and development of neurodegenerative disorders.

For a general reference to PKs malfunctioning or disregulation, see, for instance, Current Opinion in Chemical Biology 1999, 3, 459 - 465.

AKT also known as protein kinase B (PKB), and its gene family products, has been identified as a serine/ threonine protein kinase (Proc. Natl. Acad. Sci. 2001, 98, 10983- 10985; J. Cell. Sci. 2001, 114, 2903- 2910; Circ. Res. 2000, 86, 15- 23) . Three isoforms of PKB are currently known, PKB$\alpha$ (AKT1), PKB$\beta$, (AKT2), and PKB$\gamma$ (AKT3) (Proc. Natl. Acad. Sci. 1992, 89, 9267- 9271; J. Biol. Chem. 1999, 274, 9133- 9136) . PKB mediates many effects of IGF- 1 and other growth factors on tumor growth and inhibition of apoptosis *(*Cell. Signal. 2002, 14, 381- 395) . PKB plays an important role in cell proliferation, apoptosis and response to insulin. For these reasons, modulation of PKBs is of interest in the treatment of tumorigenesis, abnormal cell proliferation and diabetes.

The antiapoptotic function of PKB is reported to be mediated by its ability to phosphorylate apoptosis regulatory molecules including BAD, caspase 9, IKK-, and the forkhead transcriptional factor FKHRL1. PKB signal is also implicated in the physiological regulation of organ size *(*Nat. Cell. Biol. 1999, 1, 500- 506), glucose homeostasis *(*J. Biol. Chem. 1999, 274, 1865- 1868), vasomotor tone *(*J. Clin. Invest. 1999, 106, 493- 499), and angiogenesis *(*Nat. Med. 2000, 6, 1004- 1010) .

Manifestations of altered PKB regulation appear in both injury and disease, the most important role being in cancer. PKB kinase activity is constitutive activated in tumors with PTEN mutation, PI3- kinase mutation and overexpression, and receptor tyrosin kinase overexpression. PKB is also a mediator of normal cell functions in response to growth factor signalling. Expression of the AKT gene was found to be amplified in 15 % of human ovarian carcinoma cases *(*Proc. Natl. Acad. Sci. 1992 , 89, 9267- 9271) . AKT is also overexpressed in 12 % of pancreatic cancers *(*Proc. Natl. Acad. Sci 1996, 93, 3636- 3641) . In particular, AKT- 2 is over- expressed in 12 % of ovarian carcinomas and in 50 % of undifferentiated tumors, suggesting that PKB may be associated with tumor aggressiveness (Int. J. Cancer 1995, 64, 280- 285) . PKB is also a mediator of normal cell functions *(*Nature 1999, 401, 33- 34; Oncogene 2000, 19, 2324- 2330; Neurosci. 2000, 20, 2875- 2886) .

Elucidation of the role of PKB in the increase of growth and inhibition of apoptosis is complicated by the many protein substrates of PKB, including BAD, Forkhead (FOXO family), GSK3, Tuberin (TSC2), p27 Kip1, p70S6K, protein kinase C-, forkhead in rhabdomyosarcoma, Raf, cAMP-response element-binding protein, GSK-3, m-TOR, and the androgen receptor (Proc. Natl. Acad. 2001, 98, 7200-7205; Nature 2001, 411, 355-365; Nat. Rev. Cancer 2002, 2, 489-501).

The various PKBs vary in their abundance in different mammalian cell types. For example, PKB$\beta$ are especially abundant in highly insulin-responsive tissues, including brown fat. Modulation of PKB by small molecules can be achieved by identifying compounds that bind to and activate or inhibit one or more PKBs.

## SUMMARY OF THE INVENTION

It has been found that compounds of formula (I) described below, are kinase inhibitors and are thus useful in therapy as antitumor agents. Accordingly, a first object of the present invention is to provide a substituted 1H- pyrazolo [3, 4- b] pyridine- 3- yl- acrylamide compound represented by formula (I) :

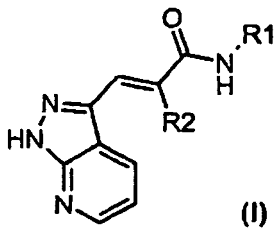

(I)

wherein:

**R1** is hydrogen or an optionally substituted group selected from straight or branched $C_1$-$C_6$ alkyl, $C_3$-$C_6$ cycloalkyl, $C_3$-$C_6$ cycloalkyl-alkyl, heterocycloalkyl, heterocycloalkyl-alkyl, aryl, arylalkyl, heteroaryl, heteroarylalkyl, aminoalkyl, hydroxyalkyl, alkoxyalkyl and a group of formula (II):

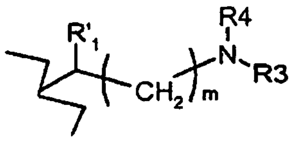

(II) ;

**R2** is hydrogen or optionally substituted aryl or heteroaryl;
**R'$_1$,** is hydrogen or an optionally substituted group selected from straight or branched $C_1$-$C_6$ alkyl, $C_3$-$C_6$ cycloalkyl, $C_3$-$C_6$ cycloalkyl-alkyl, heterocycloalkyl, heterocycloalkyl-alkyl, aryl, arylalkyl, heteroaryl and heteroarylalkyl;
**m** is 0, 1 or 2;
**R3** and **R4** are, each independently, hydrogen or an optionally substituted group selected from straight or branched $C_1$-$C_6$ alkyl and $C_3$-$C_6$ cycloalkyl, or **R3** and **R4,** taken together with the nitrogen atom to which they are bonded, may form an optionally substituted heterocycloalkyl group, optionally containing an additional heteroatom selected from N, O and S;

or isomers, tautomers, solvates, hydrates or pharmaceutically acceptable salts thereof.

Also disclosed is a method for treating diseases caused by and/or associated with dysregulated protein kinase activity, particularly one or more isoforms of the serin/ threonine kinase AKT, PLK family, protein kinase C in different isoforms, Met, PAK- 4, PAK- 5, ZC- 1, STLK- 2, DDR- 2, Aurora 1, Aurora 2, Bub- 1, Chk1, Chk2, HER2, raf1, MEK1, MAPK, EGF- R, PDGF- R, FGF- R, IGF- R, ALK , PI3K, weel kinase, Src, Abl, MAPK, ILK, MK- 2, IKK- 2, Cdc7, Nek, Cdk/ cyclin kinase family, CK2, GSK3, SULU, PDK, RET, KIT, LCK, TRKA, PKA$\alpha$, SGK1, SULU1, ERK2, CK2, VEGFR3, PDGFR, more particulary one or more isoforms of the serin/ threonine kinase AKT, which comprises administering to a mammal in need thereof an effective amount of a substituted 1H- pyrazolo [3, 4- b] pyridine- 3- yl- acrylamide compound represented by formula (I) as defined above.

Also disclosed is a method to treat a disease caused by and/or associated with dysregulated protein kinase activity selected from the group consisting of cancer and cell proliferative disorders.

Also disclosed is a method to treat specific a variety of cancers including, but not limited to: carcinoma such as bladder, breast, colon, kidney, liver, lung, including small cell lung cancer, esophagus, gall- bladder, ovary, pancreas, stomach, cervix, thyroid, prostate, and skin, including squamous cell carcinoma; hematopoietic tumors of lymphoid lineage, including leukemia, acute lymphocitic leukemia, acute lymphoblastic leukemia, B- cell lymphoma, T- cell- lymphoma, Hodgkin's lymphoma, non- Hodgkin's lymphoma, hairy cell lymphoma and Burkitt's lymphoma; hematopoietic tumors of myeloid lineage, including acute and chronic myelogenous leukemias, myelodysplastic syndrome and promyelocytic leukemia; tumors of mesenchymal origin, including fibrosarcoma and rhabdomyosarcoma; tumors of the central and peripheral nervous system, including astrocytoma, neuroblastoma, glioma and schwannomas; other tumors, including melanoma, seminoma, teratocarcinoma, osteosarcoma, xeroderma pigmentosum, keratoxanthoma, thyroid follicular cancer and Kaposi's sarcoma. Another preferred method of the present invention, is to treat cell proliferative disorders such as, but not restricted to, benign prostate hyperplasia, familial adenomatosis polyposis, neuro- fibromatosis, psoriasis, vascular smooth cell proliferation associated with atherosclerosis, pulmonary fibrosis, arthritis, glomerulonephritis, post-surgical stenosis and restenosis and Alzheimer's disease.

Also disclosed is a method for tumor angiogenesis and metastasis inhibition.

The present invention also provides methods of synthesizing the substituted 1H- pyrazolo [3, 4- b] pyridine- 3-yl- acrylamide derivatives of formula (I) prepared through a process consisting of standard synthetic transformations.

The present invention also provides a pharmaceutical composition comprising one or more compounds of formula (I) or a pharmaceutically acceptable salt thereof and a pharmaceutically acceptable excipient, carrier or diluent.

The present invention further provides a pharmaceutical composition comprising a compound of formula (I) in combination with one or more chemotherapeutic agents or radiotherapy. Such agents can include, but are not limited to, cytostatic or cytotoxic agents, antibiotic-type agents, alkylating agents, antimetabolite agents, hormonal agents, antihormonal agents such as antiestrogens, antiandrogens and aromatase inhibitors, immunological agents, interferon-type agents, cyclooxygenase inhibitors (e.g. COX-2 inhibitors), matrixmetalloprotease inhibitors, telomerase inhibitors, kinase inhibitors, anti-growth factor receptor agents, anti-HER agents, anti-EGFR agents, anti-angiogenesis agents (e.g. angiogenesis inhibitors), farnesyl transferase inhibitors, ras-raf signal transduction pathway inhibitors, cell cycle inhibitors, other cdks inhibitors, tubulin binding agents, topoisomerase I inhibitors, topoisomerase II inhibitors, agents that target microtubules, platin-based agents, DNA damaging or intercalating agents, inhibitors of kinesins, therapeutic monoclonal antibodies, inhibitors of mTOR, histone deacetylase inhibitors, and inhibitors of hypoxic response.

## DETAILED DESCRIPTION OF THE INVENTION

Indole- and azaindole- containing amide as antagonists of TGF- β are described in WO2003037862 in the name of Nippon Shinyaku Co., Japan.

Indazolylacrylamide derivatives as SGK-1 inhibitors are disclosed in WO 2005011681 in the name of Smithkline Beecham Corp.

Azaindolacrylamide derivatives as kinase inhibitors are disclosed in WO200198299 in the name of Pharmacia.

Unless otherwise specified, when referring to the compounds of formula (I) per se as well as to any pharmaceutical composition thereof or to any therapeutic treatment comprising them, the present invention includes all of the isomers, tautomers, carriers, complexes, metabolites, prodrugs, solvates, hydrates and pharmaceutically acceptable salts of the compounds of this invention.

If a chiral center or another form of an isomeric center is present in a compound of the present invention, all forms of such isomer or isomers, including enantiomers and diastereomers, are intended to be covered herein. Compounds containing a chiral center may be used as a racemic mixture, an enantiomerically enriched mixture, or the racemic mixture may be separated using well-known techniques and an individual enantiomer may be used alone. In cases in which compounds have unsaturated carbon-carbon double bonds, both the cis (Z) and trans (E) isomers are within the scope of this invention.

In cases wherein compounds may exist in tautomeric forms, such as keto-enol tautomers, each tautomeric form is contemplated as being included within this invention whether existing in equilibrium or predominantly in one form.

A metabolite of a compound of formula (I) is any compound into which this same compound of formula (I) is converted *in vivo,* for instance upon administration to a mammal in need thereof. Typically, without however representing a limiting example, upon administration of a compound of formula (I), this same derivative may be converted into a variety of compounds, for instance including more soluble derivatives like hydroxylated derivatives, which are easily excreted. Hence, depending upon the metabolic pathway thus occurring, any of these hydroxylated derivatives may be regarded as a metabolite of the compounds of formula (I).

Prodrugs are any covalently bonded compounds, which release in vivo the active parent drug according to formula (I).

The general terms as used herein, unless otherwise specified, have the meaning reported below.

The term "straight or branched $C_1$-$C_6$ alkyl" refers to a saturated aliphatic hydrocarbon radical, including straight chain and branched chain groups of from 1 to 6 carbon atoms, such as methyl, ethyl, propyl, 2-propyl, n-butyl, iso-butyl, tert-butyl, pentyl and the like. An alkyl group may be substituted or unsubstituted.

The term "$C_3$-$C_6$ cycloalkyl" refers to a 3- to 6-membered all-carbon monocyclic ring, which may contain one or more double bonds but does not have a completely conjugated π-electron system. Examples of cycloalkyl groups, without limitation, are cyclopropanyl, cyclobutanyl, cyclopentanyl, cyclopentenyl, cyclohexanyl, cyclohexenyl and cyclohexadienyl. A cycloalkyl group may be substituted or unsubstituted.

The term "heterocycloalkyl" refers to a 3- to 7-membered, saturated or partially unsaturated carbocyclic ring where one or more carbon atoms are replaced by heteroatoms such as nitrogen, oxygen and sulfur. Not limiting examples of heterocycloalkyl groups are, for instance, oxiranyl, aziridinyl, oxetanyl, azetidinyl, tetrahydrofuranyl, dihydrofuranyl, tetrahydrothiophenyl, dihydrothiophenyl, pyrrolidinyl, dihydropyrrolyl, pyranyl, dihydropyranyl, tetrahydropyranyl, tetrahydrothiopyranyl, piperidinyl, pyrazolinyl, isoxazolidinyl, isoxazolinyl, thiazolidinyl, thiazolinyl, isothiazolinyl, dioxanyl, piperazinyl, morpholinyl, thiomorpholinyl, examethyleneiminyl, homopiperazinyl and the like. A heterocycloalkyl group may be substituted or unsubstituted.

The term "aryl" refers to any aromatic carbocyclic ring system of 1 or 2 ring moieties, either fused or linked to each other through a single bond, for instance including phenyl, α - or β-naphthyl or biphenyl groups. An aryl group may

be substituted or unsubstituted.

The term "heteroaryl" refers to any aromatic heterocyclic ring which may comprise an optionally benzocondensed 5 or 6 membered heterocycle with from 1 to 3 heteroatoms selected among N, O or S.

Non limiting examples of heteroaryl groups according to the invention may thus include, for instance, pyridyl, pyrazinyl, pyrimidinyl, pyridazinyl, indolyl, imidazolyl, thiazolyl, isothiazolyl, pyrrolyl, phenyl- pyrrolyl, furyl, phenyl- furyl, oxazolyl, isoxazolyl, pyrazolyl, thienyl, benzothienyl, isoindolinyl, benzoimidazolyl, quinolinyl, isoquinolinyl, 1, 2, 3- triazolyl, 1- phenyl- 1, 2, 3- triazolyl, 2, 3- dihydroindolyl, 2, 3- dihydrobenzofuranyl, 2, 3- dihydrobenzothiophenyl; benzopyranyl, 2, 3- dihydrobenzoxazinyl, 2, 3- dihydroquinoxalinyl and the like. An heteroaryl group may be substituted or unsubstituted.

According to the present invention and unless otherwise provided, any of the above $R_1$, $R_2$, $R'_1$, $R_3$ and $R_4$ group may be optionally substituted, in any of their free positions, by one or more groups, for instance 1 to 3 groups, preferably one or two, independently selected from: $C_1$-$C_6$ alkyl, halogen, trifluoromethyl, alkylthio, cyano, nitro, formyl, alkylcarbonyl, alkylsulfonyl, carboxy, carboxamido, monoalkylcarboxamido, dialkylcarboxamido, hydroxyalkyl. In their turn, whenever appropriate, each of the above substituent may be further substituted by one or more of the aforementioned groups.

In this respect, with the term "amino" we intend the group $-NH_2$.

With the term "hydroxy" we intend the group -OH.

With the term "alkoxy" we intend any of the above $C_1$-$C_6$ alkyl groups linked to the rest of the molecule through a oxygen atom (-O-), such as methoxy, ethoxy, n-propoxy, isopropoxy and the like.

With the term "halogen" we intend a fluorine, chlorine, bromine or iodine atom.

With the term "trifluoromethyl" we intend $-CF_3$.

With the term "cyano" we intend a -CN residue.

With the term "nitro" we intend a $-NO_2$ group.

With the term "formyl" we intend a CHO group.

With the term "carboxy" we intend a COOH group.

It is clear to the skilled person that any group which name is a composite name such as, for instance, arylalkyl has to be intended as conventionally construed by the parts from which it derives, e.g. by an alkyl group which is further substituted by aryl, wherein alkyl and aryl are as above defined. Examples of aryalkyl are benzyl ($- CH_2Ph$), phenetyl ($-CH_2CH_2Ph$) and the like.

For instance, the terms "monoalkylcarboxamido" or "dialkylcarboxamido" indicate a carboxamido group where one or both hydrogens are substituted by an alkyl group. Examples of monoalkylcarboxamido are methylcarboxamido ($- CONHCH_3$), ethylcarboxamido ($- CONHCH_2CH_3$), and the like. Examples of dialkylcarboxamido are dimethylcarboxamido [$- CON (CH_3)_2$], diethylcarboxamido [$- CON (CH_2CH_3)_2$] and the like.

From all of the above it is clear to the skilled person that any of the terms such as, for instance, $C_3$-$C_6$ cycloalkyl-alkyl, heterocycloalkyl-alkyl, heteroarylalkyl, aminoalkyl, hydroxyalkyl, alkoxyalkyl, alkylthio, alkylcarbonyl, alkylsulfonyl and carboxamido, are construed by the parts from which they respectively derive.

Typically, the salts of the present invention are pharmaceutically acceptable salts. The term "pharmaceutically acceptable salt" of compounds of formula (I) refers to non- toxic salts of the compounds of this invention that retain the biological effectiveness and properties of the parent compounds.

These salts can be prepared in situ during the final isolation and purification of the compounds of formula I, or by separately reacting the base functions with a suitable organic or inorganic acid. Representative salts include, but are not limited to, the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glutamate, glucoheptanoate, glycerophosphate, hemisulfate, heptanoate, hexanoate, fumarate, hydrochloride, hydrobromide, hydroiodide, 2-hydroxyethanesulfonate, lactate, laurate, maleate, malonate, mandelate, methanesulfonate, nicotinate, 2-napthalenesulfonate, oxalate, palmitate, panthotenate, palmoate, pectinate, persulfate, 3-phenylpropionate, picrate, pivalate, propionate, salicilate, stearate, succinate, sulfate, tannate, tartrate, thiocyanate, p-toluenesulfonate and undecanoate and valerate.

Examples of acids that may be employed to form pharmaceutically acceptable acid addition salts include such inorganic acids as hydrochloric acid, sulfuric acid, nitric acid, hydrobromic acid, perchloric acid and phosphoric acid and such organic acids as oxalic acid, maleic acid, methanesulfonic acid, succinic acid and citric acid, acetic acid, trifluoroacetic acid, propionic acid, glycolic acid, lactic acid, malonic acid, malic acid, tartaric acid, benzoic acid, cinnamic acid, mandelic acid, isethionic acid and salicylic acid.

Salts formed when an acidic proton present in a compound of formula (I) are either replaced by a metal ion, e.g., an alkali metal ion such as sodium or potassium, or an alkaline earth ion such as calcium or magnesium, or coordinates with an organic base such as ethanolamine, diethanolamine, triethanolamine, tromethamine, N-methylglucamine, and the like. In other words, the basic nitrogen-containing groups can be quaternized with such agents as alkyl halides, such as methyl, ethyl, propyl, and butyl chloride, bromides, and iodides, dialkyl sulfates like dimethyl, diethyl,

dibutyl, and diamyl sulfates, long chain halides such as decyl, lauryl, myristyl and stearyl chlorides, bromides and iodides, arylalkyl halides like benzyl and phenethyl bromides, and others. Water or oil-soluble or dispersible products are thereby obtained.

Preferred compounds of formula (I) are the compounds wherein:

**R1** is hydrogen or an optionally substituted group selected from aryl, arylalkyl, heteroaryl, heteroarylalkyl, aminoalkyl, hydroxyalkyl, alkoxyalkyl and a group of formula (II) as defined above;
**R2** is hydrogen or optionally substituted heteroaryl.

Other preferred compounds of formula (I) are the compounds wherein:

**R1** is hydrogen or an optionally substituted group selected from arylalkyl, heteroarylalkyl and a group of formula (II) as defined above.

Further preferred compounds of formula (I) are the compounds wherein:

**R1** is hydrogen or an optionally substituted group selected from arylalkyl, heteroarylalkyl and a group of formula (II) as defined above, wherein **R'$_1$** is an optionally substituted group selected from aryl, arylalkyl, heteroaryl and heteroarylalkyl.

Particularly preferred compounds of formula (I) are the compounds wherein:

**R1** is hydrogen or a group of formula (II) as defined above, wherein **R'$_1$** is an optionally substituted group selected from aryl, arylalkyl, heteroaryl and heteroarylalkyl, m is 1 and **R3** and **R4** are hydrogen or **R3** and **R4,** taken together with the nitrogen atom to which they are bonded, may form an optionally substituted heterocycloalkyl group, optionally containing an additional heteroatom selected from N, O and S.

More particularly preferred compounds of formula (I) are the compounds wherein:

**R1** is hydrogen or a a group of formula (II) as defined above, wherein **R'$_1$** is an optionally substituted group selected from aryl, arylalkyl, heteroaryl and heteroarylalkyl, m is 1 and **R3** and **R4** are hydrogen;
**R2** is hydrogen or a group of formula (III):

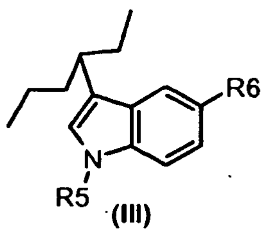

**(III)**

wherein :

**R5** is hydrogen, an optionally substituted straight or branched $C_1$-$C_6$ alkyl, alkylamino or aminoalkyl;
**R6** is hydrogen, halogen or an optionally substituted straight or branched $C_1$-$C_6$ alkyl.

Most particularly preferred compounds of formula (I) are the compounds listed below:

1. (2*E*)- 3- (1*H*- pyrazolo [3, 4- *b*] pyridin- 3- yl) acrylamide;
2. (2*E*)- N- methy- 3- (1H- pyrazolo [3, 4- b] pyridin- 3- yl) acrylamide;
3. (2*E*)- N- (2- morpholin- 4- ylethyl)- 3- (1H- pyrazolo [3, 4- b] pyridin- 3- yl) acrylamide;
4. (2*E*)- N- (2- methoxyethyl)- 3- (1H- pyrazolo [3, 4- b] pyridin- 3- yl) acrylamide;
5. (2*E*)- N- (2- pyrrolidin- 4- ylethyl)- 3- (1H- pyrazolo [3, 4- b] pyridin- 3- yl) acrylamide;
6. (2*E*)- N- benzyl- 3- (1H- pyrazolo [3, 4- b] pyridin- 3- yl) acrylamide;
7. (2*E*)- N- (2- chlorobenzyl)- 3- (1H- pyrazolo [3, 4- b] pyridin- 3- yl) acrylamide;
8. (2*E*)- N- (3- chlorobenzyl)- 3- (1H- pyrazolo [3, 4- b] pyridin- 3- yl) acrylamide;

9. (2*E*)- N- (4- chlorobenzyl)- 3- (1H- pyrazolo [3, 4- b] pyridin- 3- yl) acrylamide;
10. (2*E*)- N- (3, 4- dichlorobenzyl)- 3- (1H- pyrazolo [3, 4- b] pyridin- 3- yl) acrylamide;
11. (2*E*)- N- (2- fluorobenzyl)- 3- (1H- pyrazolo [3, 4- b] pyridin- 3- yl) acrylamide;
12. (2*E*)- N- (3- fluorobenzyl)- 3- (1H- pyrazolo [3, 4- b] pyridin- 3- yl) acrylamide;
13. (2*E*)- N- (4- fluorobenzyl)- 3- (1H- pyrazolo [3, 4- b] pyridin- 3- yl) acrylamide;
14. (2*E*)- N- (3, 4- difluorobenzyl)- 3- (1H- pyrazolo [3, 4- b] pyridin- 3- yl) acrylamide;
15. (2*E*)- N- (2, 4- difluorobenzyl)- 3- (1H- pyrazolo [3, 4- b] pyridin- 3- yl) acrylamide;
16. (2*E*)- N- (2, 5- difluorobenzyl)- 3- (1H- pyrazolo [3, 4- b] pyridin- 3- yl) acrylamide;
17. (2*E*)- N- (2, 6- difluorobenzyl)- 3- (1H- pyrazolo [3, 4- b] pyridin- 3- yl) acrylamide;
18. (2*E*)- N- (2- methoxybenzyl)- 3- (1H- pyrazolo [3, 4- b] pyridin- 3- yl) acrylamide;
19. (2*E*)- N- (3- methoxybenzyl)- 3- (1H- pyrazolo [3, 4- b] pyridin- 3- yl) acrylamide;
20. (2*E*)- N- (4- methoxybenzyl)- 3- (1H- pyrazolo [3, 4- b] pyridin- 3- yl) acrylamide;
21. (2*E*)- N- (4- ethoxybenzyl)- 3- (1H- pyrazolo [3, 4- b] pyridin- 3- yl) acrylamide;
22. (2*E*)- N- (4- methoxybenzyl)- 3- (1H- pyrazolo [3, 4- b] pyridin- 3- yl) acrylamide;
23. (*E*)- N- ((S)- 2- amino- 1- benzyl- ethyl)- 3- (1*H*- pyrazolo [3, 4- *b*] pyridin- 3- yl) acrylamide;
24. (2*E*)- *N*- [(2*S*)- 2- amino- 3- phenylpropyl]- 3- (1*H*- pyrazolo [3, 4- *b*] pyridin- 3- yl) acrylamide;
25. (2*E*)- *N*- [(1*S*)- 2- amino- 1- (1*H*- indol- 3- ylmethyl) ethyl]- 3- (1*H*- pyrazolo [3, 4- *b*] pyridin- 3- yl) acrylamide;
26. (2*E*)- *N*- [(1*S*)- 2- amino- 1- (1- naphthylmethyl) ethyl]- 3- (1*H*- pyrazolo [3, 4- *b*] pyridin- 3- yl) acrylamide;
27. (2*E*)- *N*- [(1*S*)- 2- amino- 1- (2- naphthylmethyl) ethyl]- 3- (1*H*- pyrazolo [3, 4- *b*] pyridin- 3- yl) acrylamide;
28. (2*E*)- *N*- [(1*S*)- 1- phenyl- 2- pyrrolidin- 1- ylethyl]- 3- (1*H*- pyrazolo [3, 4- *b*] pyridin- 3- yl) acrylamide;
29. (2*E*)- *N*- [(1*S*)- 2- amino- 1- phenylethyl]- 3- (1*H*- pyrazolo [3, 4- *b*] pyridin- 3- yl) acrylamide
30. (2*E*)- *N*- [(1*S*)- 3- amino- 1- benzylpropyl]- 3- (1*H*- pyrazolo [3, 4- *b*] pyridin- 3- yl) acrylamide;
31. (2*E*)- N- [(1S)- 2- amino- 1- (thien- 2- ylmethyl) ethyl]- 3- (1H- pyrazolo [3, 4- b] pyridin- 3- yl) acrylamide;
32. (2*E*)- N- [(1S)- 2- morpholin- 4- yl- 1- phenylethyl]- 3- (1H- pyrazolo [3, 4- b] pyridin- 3- yl) acrylamide;
33. *N*α- [(2*E*)- 3- (1*H*- pyrazolo [3, 4- *b*] pyridin- 3- yl) prop- 2- enoyl]- L- phenylalaninamide;
34. (2*E*)- *N*- [(1*S*)- 2- (4- methylpiperazin- 1- yl)- 1- phenylethyl]- 3- (1*H*- pyrazolo [3, 4- *b*] pyridin- 3- yl) acrylamide;
35. (2*E*)- 2- (5- bromo- 1- methyl- 1H- indol- 3- yl)- N- [(1S)- 2- (4- methylpiperazin- 1- yl)- 1- phenylethyl]- 3- (1H- pyrazolo [3, 4- b] pyridin- 3- yl) acrylamide;
36. (2*E*)- N- [(1*S*)- 2- amino- 1- benzylethyl]- 2- (5- bromo- 1- methyl- 1H- indol- 3- yl)- 3- (1H- pyrazolo [3, 4- b] pyridin- 3- yl) acrylamide;
37. (2*E*)- 2- (5- bromo- 1- methyl- 1H- indol- 3- yl)- 3- (1H- pyrazolo [3, 4- b] pyridin- 3- yl) acrylamide;
38. (2*E*)- 2- (5- bromo- 1*H*- indol- 3- yl)- *N*- [(1*S*)- 2- (4- methylpiperazin- 1- yl)- 1- phenylethyl]- 3- (1*H*- pyrazolo [3, 4- *b*] pyridin- 3- yl) acrylamide;
39. (2*E*)- 2- (1, 5- dimethyl- 1*H*- mdol- 3- yl)- *N*- [(1*S*)- 2- (4- methylpiperazm- 1- yl)- 1- phenylethyl]- 3- (1*H*- pyrazolo [3, 4- *b*] pyridin- 3- yl) acrylamide;
40. (2*E*)- 2- (5- ethyl- 1- methyl- 1*H*- indol- 3- yl)- *N*- [(1*S*)- 2- (4- methylpiperazin- 1- yl)- 1- phenylethyl]- 3- (1*H*- pyrazolo [3, 4- *b*] pyridin- 3- yl) acrylamide;
41. (2*E*)- *N*- benzyl- 2- (5- bromo- 1- methyl- 1*H*- indol- 3- yl)- 3- (1*H*- pyrazolo [3, 4- *b*] pyridin- 3- yl) acrylamide;
42. (2*E*)- 2- (5- bromo- 1- methyl- 1*H*- indol- 3- yl)- *N*- [2- (dimethylamino) ethyl]- 3- (1*H*- pyrazolo [3, 4- *b*] pyridin- 3- yl) acrylamide;
43. (2*E*)- 2- (5- bromo- 1- methyl- 1*H*- indol- 3- yl)- *N*- (2- morpholin- 4- ylethyl)- 3- (1*H*- pyrazolo [3, 4- *b*] pyridin- 3- yl) acrylamide;
44. (2*E*)- 2- phenyl- 3- (1*H*- pyrazolo [3, 4- *b*] pyridin- 3- yl) acrylamide;
45. (2*E*)- 2- (3- bromophenyl)- 3- (1*H*- pyrazolo [3, 4- *b*] pyridin- 3- yl) acrylamide;
46. (2*E*)- 3- (1*H*- pyrazolo [3, 4- *b*] pyridin- 3- yl)- 2- [3- (trifluoromethyl) phenyl] acrylamide;
47. (2*E*)- 2- (3- bromophenyl)- *N*- [(1*S*)- 2- (4- methylpiperazin- 1- yl)- 1- phenylethyl]- 3- (1*H*- pyrazolo [3, 4- *b*] pyridin- 3- yl) acrylamide;
48. (2*E*)- *N*- [(1*S*)- 2- (4- methylpiperazin- 1- yl)- 1- phenylethyl]- 3- (1*H*- pyrazolo [3, 4- *b*] pyridin- 3- yl)- 2- [3- (trifluoromethyl) phenyl] acrylamide;
49. (2*E*)- *N*- [(1*S*)- 2- (4- methylpiperazin- 1- yl)- 1- phenylethyl]- 2- phenyl- 3- (1*H*- pyrazolo [3, 4- *b*] pyridin- 3- yl) acrylamide and
50. (2*E*)- 2- (3- chlorophenyl)- *N*- [(1*S*)- 2- (4- methylpiperazin- 1- yl)- 1- phenylethyl]- 3- (1*H*- pyrazolo [3, 4- *b*] pyridin- 3- yl) acrylamide.

For a reference to any specific compound of formula (I) of the invention, optionally in the form of a pharmaceutically acceptable salt, see the experimental section and claims.

The present inventions also provides a process for the preparation of compounds of formula (I) as defined above, characterized in that the process comprises:

e) deprotecting a compound of formula (VIII):

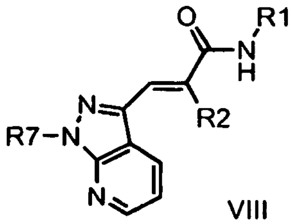

VIII

wherein **R1** and **R2** are as defined above, and **R7** is benzyl, 4-methoxybenzyl, 2,4-dimethoxybenzyl or β-tosyl ethyl; optionally separating the resulting compound into the single isomers; converting the resulting compound of formula (I) into a different compound of formula (I) and/or into a pharmaceutically acceptable salt if desired.

The present invention further provides a process for the preparation of a compound of formula (I) as defined above, characterized in that the compound of formula (VIII) as defined above is prepared according to the following steps:

a) protecting the compound of formula (IV):

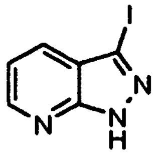

IV

with an alkylating compound of formula **R7-Z,** wherein **R7** is as defined above and Z is halogen or a suitable leaving group;

b) reacting the resulting compound of formula (V) :

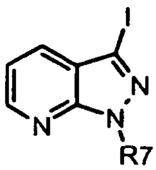

V

with an alkenyl derivative of formula (XIII) :

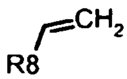

XIII

wherein **R8** is- COO ($C_1$- $C_6$) Alkyl, -COOH, $-CONH_2$, -CN or- $CH_2OH$;

c) reacting the resulting compound of formula (VI):

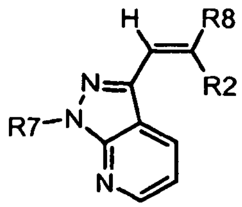

; VI

wherein **R2** is hydrogen and **R7** and **R8** are as defined above;
d) reacting the resulting compound of formula (VII):

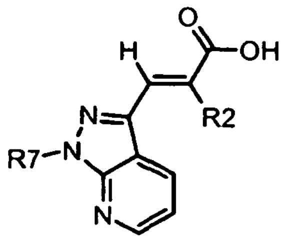

VII

wherein **R2** is hydrogen and **R7** is as defined above, with an amine compound of the formula **R1-NH$_2$,** wherein **R1** is as defined above, to give a compound of formula (VIII) wherein **R2** is hydrogen and **R1** and **R7** are as defined above.

The present invention further provides a process for the preparation of a compound of formula (I) as defined above, characterized in that the compound of formula (VIII) as defined above is prepared according to the following steps:

f) reacting the compound of formula (V) as defined above, with carbon monoxide or a suitable succedaneous that generate carbon monoxide, to give a compound of formula (IX);
g) reacting the resulting compound of formula (IX) :

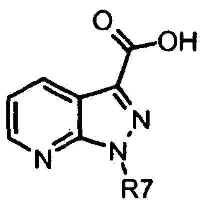

IX

wherein **R7** is as defined above, with O, N- Dimethyl- hydroxylamine (Weinreb amine) ;
h) reducing the resulting compound of formula (X):

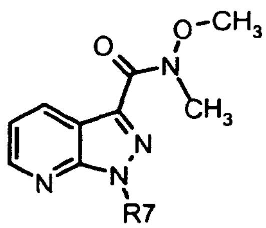

X

wherein **R7** is as defined above;

i) reacting the resulting compound of formula (XI):

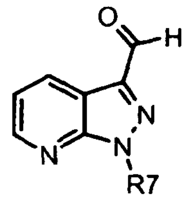

XI

wherein **R7** is as defined above, with a compound of formula (XII):

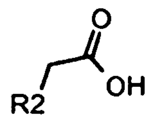

XII

wherein **R2** is Aryl or Heteroaryl ;

1) reacting the resulting compound of formula (VII):

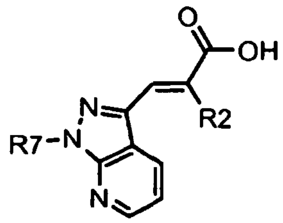

VII

wherein **R2** is Aryl or Heteroaryl and **R7** is as defined above with an amine compound of the formula **R1-NH2,** wherein **R1** is as defined above, to give a compound of formula (VIII) wherein **R2** is Aryl or Heteroaryl and **R1** and **R7** are as defined above.

A compound of formula (I) can also be transformed into a pharmaceutically acceptable salt according to standard procedures that are known to those skilled in the art.

Alternatively, a compound of formula (I) that is obtained as a salt can be transformed into the free base or the free acid according to standard procedures that are known to the skilled person. The synthesis of a compound of formula (I), according to the synthetic process described above, can be conducted in a stepwise manner, whereby each intermediate is isolated and purified by standard purification techniques, like, for example, column chromatography, before carrying out the subsequent reaction.

According to the step a) of the process, the protection of a compound of formula (IV) into a compound of formula (V) can be accomplished in a variety of ways and experimental conditions, which are widely known in the art for the insertion of a protecting group. For example, when R7 is a p-methoxy-benzyl group the reaction can be carried out with p-methoxy-benzyl chloride in the presence of a base such as potassium hydroxide, potassium carbonate, caesium carbonate in a suitable solvent such as tetrahydrofuran, 1,4 dioxane, N,N-dimethylformamide at a temperature ranging from room temperature to reflux, for a time ranging from 30 min. to about 24 hours.

Alternatively, when R7 is the β- tosylethyl (TSE) group the protection can be carried out by reaction with 1-Ethenesulfonyl- 4- methyl- benzene, in the presence of a inorganic base such as sodium carbonate, potassium carbonate in a suitable solvent such as tetrahydrofuran, 1, 4 dioxane, N, N- dimethylformamide at a temperature ranging from room

temperature to reflux, for a time ranging from 30 min. to about 24 hours.

According to the step b) of the process, the transformation of the compound of formula (V) into a compound of formula (VI) is accomplished by reacting the compound of formula (V) with alkenyl derivatives in a variety of ways and experimental conditions, which are widely known in the art as Heck reaction. Preferably, this reaction is carried out with ethyl acrylate in the presence of a palladium catalyst such as palladium acetate, with a phosphine such as tris (o- tolyl)-phosphine in presence of potassium carbonate, or of a tertiary amine such as triethylamine (TEA), and in a suitable solvent such as tetrahydrofuran, 1, 4- dioxane, acetonitrile at reflux for a time varying from about 30 min. to about 24 hours.

According to the step c) of the process, the hydrolysis of the compound of formula (VI) can be accomplished in a variety of ways and experimental conditions, which are widely known in the art as ester hydrolysis. As an example, this reaction may be carried out under basic conditions, for instance in the presence of an inorganic base such as sodium hydrate or potassium hydrate or lithium hydrate in a suitable solvent such as ethanol or a mixture of THF methanol and water at room temperature for a time ranging from 1 to 24 hours.

According to the step d) of the process, the transformation of a compound of formula (VII) into a compound of formula (VIII) can be accomplished in a variety of ways and experimental conditions, which are widely known in the art for the preparation of amides. The reaction of the carboxylic acid compound of formula (VII) with amines and in particular the compounds of formula (II) can be carried out in the presence of a suitable condensing agent such as 2- (1H-benzotriazol- 1- yl)- 1, 1, 3, 3- tetramethyluronium tetrafluoroborate (TBTU), soluble or polymer supported 1, 3- dicyclohexylcarbodiimide (DCC), 1, 3- diisopropylcarbodiimide (DIC), 1- (3- dimethylaminopropyl)- 3- ethylcarbodiimide (EDCI), 1.1'- carbonyldiimidazole (CDI), benzotriazol- 1- yloxytripyrrolidinophosphonium hexafluorophosphate (PyBOP), in a suitable solvent such as, for instance, dichloromethane, chloroform, tetrahydrofuran, diethyl ether, 1, 4- dioxane, acetonitrile, toluene, dimethylacetamide (DMA), N- methylpyrrolidone (NMP), or N, N- dimethylformamide (DMF), at a temperature ranging from about- 10°C to reflux and for a suitable time, for instance from about 30 minutes to 96 hours. The said reaction is optionally carried out in the presence of a suitable catalyst, for instance 4- dimethylaminopyridine (DMAP), or in the presence of a further coupling reagent such as N- hydroxybenzotriazole (HOBT), or in the presence of a suitable base such as triethylamine (TEA) or N, N- diisopropyl- N- ethylamine (DIPEA) .

Alternatively, this same reaction can be also carried out, for example, through a mixed anhydride method, by using an alkyl chloroformate such as ethyl, iso- butyl, or isopropyl chloroformate, in the presence of a tertiary base such as triethylamine, N, N- diisopropyl- N- ethylamine or pyridine, in a suitable solvent such as, for instance, toluene, dichloromethane, chloroform, tetrahydrofuran, acetonitrile, diethyl ether, 1, 4- dioxane, or N, N- dimethylformamide, at a temperature ranging from about- 30°C to room temperature.

According to the step e) of the process, the deprotection of a compound of formula (VIII) where R7 is a protecting group of the amine moiety, can be made in a variety of ways according to conventional methods for deprotecting amino groups. Depending on the amino protecting group, this reaction can be conducted in different ways. In one aspect, such reaction can be carried out by treatment with an inorganic acid, such as hydrochloric, sulphuric or perchloric acid, or an organic acid, such as trifluoroacetic or methanesulfonic acid, in a suitable solvent, such as water, methanol, ethanol, 1, 4- dioxane, tetrahydrofuran, diethyl ether, diisopropyl ether, acetonitrile, N, N- dimethylformamide, dichloromethane or mixtures thereof, at a temperature ranging from- 10°C to 80°C, and for a period of time ranging from 30 minutes to 48 hours. In another aspect, such reaction can be carried out by treatment with an inorganic base, such as lithium or sodium or potassium hydroxide, or sodium or potassium or caesium carbonate, or with an organic base, such as sodium or potassium trt- butoxide, or triethylamine or N, N- diisopropylethylamine, or with anhydrous hydrazine or hydrazine hydrate in a suitable solvent such as water, methanol, ethanol, 1, 4- dioxane, tetrahydrofuran, diethyl ether, diisopropyl ether, acetonitrile, N, N- dimethylformamide, dichlorometane or mixtures thereof, at a temperature ranging from- 10°C to 80°C, and for a period of time ranging from 30 minutes to 72 hours. In still another option, such reaction can be carried out by treatment with hydrogen or cyclohexene or cyclohexadiene and a hydrogenation catalyst, such as palladium on carbon, or with a metal, such as zinc, and an inorganic or organic acid, such as hydrochloric or acetic acid, in a suitable solvent such as water, methanol, ethanol, 1, 4- dioxane, tetrahydrofuran or mixture thereof, at a temperature ranging from- 10°C to 80°C, and for a period of time ranging from 30 minutes to 72 hours.

The reaction of deprotection of R7, may also be efficacious in removing a further amino protective group eventually present as R3 or R4 .

According to the step f) of the process, the transformation of a compound of formula (V) into a compound of formula (IX) can be accomplished in a variety of ways and experimental conditions, which are widely known in the art as palladium hydroxycarbonylation reaction (ref. Cacchi S. et al., Org. Lett. 5, (2003) 23, 4269) . Preferably, this reaction is carried out with carbon monoxide or a suitable succedaneous that generated carbon monoxide in situ, such as lithium formate and acetic anhydride, in the presence of palladium catalyst such as palladium di- benzylidenacetone ($Pd_2dba_3$) . The said reaction is optionally carried out in the presence of a suitable catalyst, for instance lithium chloride or of a tertiary amine such as triethylamine (TEA), N, N- diisopropylethylamine (DiPEA), and in a suitable solvent such as tetrahydrofuran, 1, 4- dioxane, acetonitrile or N, N- dimethylformamide, at reflux for a time varying from about 30 min. to about 24 hours.

According to the step g) of the process, the transformation of compound of formula (IX) into a compound of formula (X) can be accomplished in a variety of ways and experimental conditions, which are widely known in the art as Weinreb amide formation.

Typically, the reaction is carried out by using a suitable activating reagent such as 2- (1H- benzotriazol- 1- yl)- 1, 1, 3, 3- tetramethyluronium tetrafluoroborate (TBTU), 1, 3- diisopropylcarbodiimide (DIC), 1- (3- dimethylaminopropyl)- 3- ethylcarbodiimide (EDCI), 1.1'- carbonyldiimidazole (CDI), benzotriazol- 1- yloxytripyrrolidinophosphonium hexafluorophosphate (PyBOP), in a suitable solvent such as, for instance, dichloromethane, chloroform, tetrahydrofuran, diethyl ether, 1, 4- dioxane, acetonitrile, toluene, dimethylacetamide (DMA), N- methylpyrrolidone (NMP), or N, N- dimethylformamide (DMF), at a temperature ranging from about- 10°C to reflux and for a suitable time, for instance from about 30 minutes to about 96 hours. Preferably the reaction may be carried out in the presence of a suitable base such as triethylamine (TEA) or N, N- diisopropyl- N- ethylamine (DIPEA), with the addition of the O, N- Dimethyl- hydroxylamine (Weinreb amine) in a mixture of DIPEA and DMF as solvent, to obtain the corresponding Weinreb amide derivative.

According to the step h) of the process, the transformation of the compound of formula (X) into a compound of formula (XI) can be accomplished in a variety of ways according to conventional methods for reducing the Weinreb amide group into an aldehyde group. As an example, this reaction may be carried out in the presence of a suitable reducing agent such as lithium aluminium hydride (LiAlH4), in a suitable solvent as tetrahydrofurane (THF), diethyl ether, 1, 4- dioxane, at a temperature ranging from about- 10°C to reflux and for a suitable time, for instance from about 1 hour to about 96 hours.

According to the step i) of the process, the transformation of the compound of formula (XI) into a compound of formula (VII) can be accomplished in a variety of ways which are widely known in the art as Perkin condensation. Preferably, this reaction is carried out by reacting the aldehyde (XI) with aryl or heteroaryl acetic acid derivative of formula (XII) in the presence of acetic anhydride and triethyl amine(TEA) which are also used as solvent at a temperature ranging from about room temperature to reflux and for a suitable time, for instance from about 1 hour to about 24 hours.

According to the step 1) of the process, the transformation of a compound of formula (VII) into a compound of formula (VIII) can be accomplished in a way analogous to that specified above under d).

Compound IV is prepared from the scaffold pyrazolo [3, 4- *b*] pyridine (Registry Number 18417- 64- 6, also described in Can.J.Chem., 66, 1988, 420- 428 ) according to the conditions reported in Tetrahedron Letters 43 (2002) 2695- 2697.

The TSE protecting group was inserted and removed according to the condition described in Tetrahedron 60 (2004) 901.

The alkenyl compounds of formula (XIII) involved in the step b) are commercial available.

The heteroaryl acetic acids of formula (XII) involved in the step i) when not commercial available can be prepared following the process described in Davis, Peter David; Hill, Christopher Huw; Lawton, Geoffrey, *Preparation of 3- (2, 5- dioxopyrrol- 3- yl) indole derivatives as protein kinase inhibitors.* Eur. Pat. Appl. (1990), EPXXDW EP 384349 A1.

Substituted pyrazolo [3, 4- *b*] pyridine derivatives can be prepared using standard procedures in organic synthesis as reported, for instance, in Smith, Michael- March's Advanced Organic Chemistry: reactions mechanisms and structure- 5th Edition, Michael B. Smith and Jerry March, John Wiley & Sons Inc., New York (NY), 2001.

It is known to the skilled person that transformation of a chemical function into another may require that one or more reactive centers in the compound containing this function be protected in order to avoid undesired side reactions. Protection of such reactive centers, and subsequent deprotection at the end of the synthetic transformations, can be accomplished following standard procedures described, for instance, in: Green, Theodora W. and Wuts, Peter G.M. - Protective Groups in Organic Synthesis, Third Edition, John Wiley & Sons Inc., New York (NY), 1999.

Those skilled in the art will recognize if a stereocenter exists in compounds of formula (I).

Accordingly, the present invention includes both possible stereoisomers and includes not only racemic compounds but the individual enantiomer as well. When a compound is desired as a single enantiomer, it may be obtained by stereospecific synthesis or by resolution of the final product or any convenient intermediate.

In cases where a compound of formula (I) contains one or more asymmetric centers, said compound can be separated into the single isomer by procedures known to those skilled in the art. Such procedures comprise standard chromatographic techniques, including chromatography using a chiral stationary phase, or crystallization. Stereochemistry of Organic Compounds by E.L.Eliel, S.H. Wilen and L.N. Mander (Wiley- Interscience, 1994), reports, for instance, general methods for separation of compounds containing one or more asymmetric centers.

The compounds of this invention may be made by a variety of methods, including standard chemistry. Any previously defined variable will continue to have the previously defined meaning unless otherwise indicated. Illustrative general synthetic methods are set out below and then the specific compounds of the invention are prepared in the Working Examples.

The compounds of this invention can be prepared from readily available starting materials using the following general methods and procedures. It will be appreciated that, where typical or preferred process conditions (*i.e.*, reaction temperatures, times, mole ratios of reactants, solvents, pressures) are given, other process conditions can also be used

unless otherwise stated. Optimum reaction conditions may vary with the particular reactants or solvent used, but one skilled in the art can determine such conditions by routine optimization procedures.

## PHARMACOLOGY

The compounds of formula (I) are active as protein kinase inhibitors and are therefore useful, for instance, to restrict the unregulated proliferation of tumor cells.
In therapy, they can be used in the treatment of various tumors, such as those formerly reported, as well as in the treatment of other cell proliferative disorders such as benign prostate hyperplasia, familial adenomatosis polyposis, neuro-fibromatosis, psoriasis, vascular smooth cell proliferation associated with atherosclerosis, pulmonary fibrosis, arthritis, glomerulonephritis, post-surgical stenosis and restenosis and Alzheimer’s disease.
The short forms and abbreviations used herein have the following meaning:

| | |
|---|---|
| Ci | Curie |
| DMSO | dimethylsulfoxide |
| ID | identity |
| KDa | kiloDalton |
| microCi | microCurie |
| mg | milligram |
| microg | microgram |
| mL | milliliter |
| microL | microliter |
| $\mu$L | microliter |
| M | molar |
| mM | millimolar |
| microM | micromolar |
| nM | nanomolar |

### Preparation of AKT 1, 2 and 3 for use in biochemical assay

The N-terminal GST fusion proteins used for the assays were prepared according to the methods described in Kumar, C. C. Biochem. Biophys. ACTA 2001, 1526, 257-268, and in Alessi, D. R.. Biochem. J. 1998 331, 299-308.

### Assays

Compounds of the present invention were tested in biochemical as well as in cell-based assays, as described below.

### Biochemical assay for inhibitors of AKT1 kinase activity

The inhibitory activity of putative kinase inhibitors and the potency of selected compounds were determined using a trans-phosphorylation assay.
A specific substrate was incubated with the kinase in appropriate buffer conditions in the presence of ATP traced with $^{33}P$- $\gamma$- ATP (gamma phosphate- labeled, Redivue™
Code Number AH9968, 1000-3000Ci/mmole, Amersham Biosciences Piscataway, NJ, USA), optimal cofactors and test compound.
At the end of the phosphorylation reaction, more than 98% cold and radioactive ATP were captured by an excess of Dowex ion exchange resin. The resin was allowed to settle to the bottom of reaction wells by gravity. Supernatant, containing substrate peptide, was subsequently withdrawn and transferred into a counting plate, and radioactivity (corresponding to phosphate incorporated into peptide) was evaluated by $\beta$-counting.

Reagent/assay conditions

i. Dowex resin preparation

500 g of wet resin (SIGMA, custom prepared resin DOWEX 1x8 200-400 mesh, 2.5 Kg) are weighed out and

diluted to 2 1 in 150 mM sodium formate, pH 3.00.

The resin is allowed to settle down (some hours) and then the supernatant is discarded.

After three washes as above over a couple of days, the resin is allowed to settle, the supernatant is discarded and two volumes of 150 mM sodium formate buffer are added per volume of pellet. The final pH was then measured and should be around 3.00.

The washed resin was kept at 4° C before use, and was stable for more than one week.

ii. Kinase Buffer (KB)

Kinase buffer was composed of 50 mM TRIS pH 7.5 containing10 mM $MgCl_2$, 1 mM DTT, 3 microM $Na_3VO_4$, and 0.2 mg/mL BSA. 3X KB is buffer of the same composition and pH as KB, but with three times the concentration of each component.

iii.Assay conditions (final concentrations) for AKT1

The kinase assay was run with a final enzyme concentration of 5 nM, in the presence of 132 microM ATP, 0.88 nM $^{33}P$- $\gamma$- ATP and 30 microM substrate, a carboxy- terminally biotinylated peptide of the following sequence: ARKRERTYSFGHHA- biotin. The peptide was obtained in batches of >95% peptide purity from PRIMM labs Inc. (Boston MA, USA) .

<u>Robotized dowex assay</u>

The "test" mixture of 15 microL consisting of:

1) 5 $\mu$l/well of 3x Enzyme mix (done in Kinase Buffer 3X),
2) 5 $\mu$l/ well of 3x substrate and ATP mix (done in ddH2O), together with $^{33}P$- $\gamma$- ATP,
3) 5 $\mu$l/well of 3x test compounds (diluted into ddH2O - 3% DMSO).

<u>Dilution of compounds and assay scheme is reported below.</u>

i. Dilution of compounds

10 mM stock solutions of test compounds in 100% DMSO were distributed into 96 well 12x8 format microtiter plates.

For % inhibition studies, dilution plates at 1 mM, 10 microM and 100 microM were prepared in 100% DMSO, then diluted to 3X final desired concentration (30, 3 and 0.3 microM) in $ddH_2O$, 3% DMSO. A Multimek 96 (Beckman Coulter, Inc. 4300 N. Harbor Boulevard, P.O. Box 3100 Fullerton, CA 92834-3100 USA) was used for compound pipetting into test plates.

For IC50 determination, starting solutions of 30 microM compound in 3% DMSO were derived from 1 mM/100% DMSO stock solutions. These 30 microM starting solutions were used for generation of a further 9 serial 1/3 dilutions in $ddH_20$, 3% DMSO, so as to generate a 10-point dilution curve at 3X the final assay concentration. Serial dilution was conducted in 96-well plates using a Biomek 2000 (Beckman Coulter) system. Dilution curves of 7 compounds/plate were prepared, and each plate also included a 10-point dilution curve of Staurosporine, as well as several negative and positive control wells.

**<u>Assay scheme</u>**

384- well plates, V bottom (test plates) are prepared with 5 $\mu$l of the compound dilution (3X) and then placed onto a PlateTrak 12 robotized station (Perkin Elmer; the robot has one 384- tips pipetting head for starting the assay plus one 96- tips head for dispensing the resin) together with one reservoir for the Enzyme mix (3X) and one for the ATP mix (3X) .

At the start of the run, the robot aspirates 5 $\mu$l of ATP mix, makes an air gap inside the tips (3 $\mu$l) and aspirates 5 $\mu$l of AKT mix. The following dispensation into the plates allows the kinase reaction to start upon 3 cycles of mixing, done by the robot itself.

At this point, the correct concentration is restored for all reagents.

The robot incubates the plates for 60 minutes at room temperature, and then stops the reaction by pipetting 70 $\mu$l of dowex resin suspension into the reaction mix. Three cycles of mixing are done immediately after the addition of the resin.

The resin suspension has to be carefully stirred during the whole step of reaction stop because its settling velocity is extremely high.

The resin suspension is very dense; in order to avoid tip clogging, wide bore tips are used to dispense it.

Another mixing cycle is performed after all the plates are stopped, this time using normal tips: the plates are then allowed to rest for about one hour in order to maximize ATP capture. At this point, 20 μl of the supernatant are transferred into 384- Optiplates (Perkin- Elmer), with 70 μl of Microscint 40 (Perkin- Elmer) ; after 5 min of orbital shaking the plates are read on a Perkin- Elmer Top Count radioactivity counter.

Data are analysed by an internally customized version of the SW package "Assay Explorer" that provides either % inhibition for primary assays or sigmoidal fittings of the ten-dilutions curves for $IC_{50}$ determination, for the secondary assays/hit confirmation routines.

**iii.** Data analysis

Data were analysed using a customized version of the "Assay Explorer" software package (Elsevier MDL, San Leandro, CA 94577). For single compound concentrations, inhibitory activity was typically expressed as % inhibition obtained in presence of compound, compared to total activity of enzyme obtained when inhibitor is omitted.

Compounds showing desired inhibition were further analysed in order to study the potency of the inhibitor through $IC_{50}$ calculation. In this case, inhibition data obtained using serial dilutions of the inhibitor were fitted by non-linear regression using the following equation:

$$v = v_0 + \frac{(v_0 - v_b)}{1 + 10^{n(\log IC_{50} - \log[I])}}$$

where $v_b$ is the baseline velocity, $v$ is the observed reaction velocity, $v_o$ is the velocity in the absence of inhibitors, and [I] is the inhibitor concentration.

**Cell-based assays for inhibitors of AKT1 kinase activity**

**Inhibition of AKT induced S6 ribosomal protein phosphorylation in MCF7**

MCF- 7 cells (ATCC# HTB- 22) were seeded in 96- well poly- lysine coated clear- and flat- bottomed black plates (Matrix Technologies Inc., Hudson, NH, USA), in E- MEM medium (MEM+ Earle's BSS + 2 mM glutamine + 0.1 mM non- essential amino acids) containing 10% of FCS at a density of 7000 cells/ well, and incubated overnight at 37°C, 5% CO2, 100% relative humidity. After this incubation, cells were treated with increasing compound doses for 4 hour at 37°C. Afterwards, cells were fixed with PBS/ 3.7% paraformaldehyde for 15 min at room temperature, wash twice with 200 μl/ well D- PBS and permeabilize with a D- PBS solution containing 0.1% Triton X- 100 (Sigma- Aldrich, St. Louis, MO, USA) and 0.1% powder Milk for 15 minutes (staining solution) . To the wells was then, add the primary anti- phospho-S6 (Ser 235/236) antibody (Cell Signaling, cat. # 2211) diluted 1/200 in staining solution (50 μl/ well) and incubate for 1 hour at 37°C.Wells were then washed twice with 200 μl/ well D- PBS and add the anti- rabbit Cy™5- conjugated (Far-red) secondary antibody (Amersham Biosciences, Little Chalfont, Buckinghamshire, UK) (1/500) in staining solution (50 μl/ well) containing 2 μg/ml DAPI (4, 6- diamidino- 2- phenylindole) and incubate for 1 hour at 37°C. Wells were then washed X2 with 200 μL/ well D- PBS, and 200 microL PBS are left in each well for immunofluorescence analysis. Fluorescence images in the DAPI and Cy5™ channels were automatically acquired, stored and analysed using a Cellomics ArrayScan™ IV instrument (Cellomics, Pittsburgh, USA) ; the Cellomics Cytotoxicity Algorithm was used to quantify cytoplasmic fluorescence associated with phospho- S6 (Cy5™ signal parameter: "Mean Lyso Mass- pH") for each cell in 10 fields/ well, and eventually expressed as a mean population value. Unless otherwise stated, reagents were obtained from Sigma- Aldrich, St. Louis, MO, USA.

**Biochemical assay for inhibitors of AKT2 kinase activity**

Assay conditions

The in vitro kinase inhibition assay was conducted in the same way as described for AKT1. The kinase assay was run with a final enzyme concentration of 10 nM, in the presence of 396 microM ATP, 0.88 nM $^{33}P$- γ- ATP and 8.5 microM substrate, a carboxy- terminally biotinylated peptide of the following sequence:

ARKRERTYSFGHHA- biotin. The peptide was obtained in batches of >95% peptide purity from PRIMM labs Inc. (Boston MA, USA) .

**Biochemical assay for inhibitors of AKT3 kinase activity**

Assay conditions

The in vitro kinase inhibition assay was conducted in the same way as described for AKT1. The kinase assay was run with a final enzyme concentration of 1 nM, in the presence of 180 microM ATP, 0.88 nM $^{33}P$- $\gamma$- ATP and 28.5 microM substrate, a carboxy- terminally biotinylated peptide of the following sequence:

ARKRERTYSFGHHA- biotin. The peptide was obtained in batches of >95% peptide purity from PRIMM labs Inc. (Boston MA, USA) .

Most compounds of formula (I) of the present invention showed $IC_{50}$ values on AKT1, AKT2, AKT3 in the low micromolar range.

In addition the selected compounds have been characterized for specificity on a panel of many other kinases, among which PKA$\alpha$, SGK1, Cdk2A, IGF1-R, Aurora-2, PLK1, SULU1, ERK2, CK2, GSK3$\beta$, PKC$\beta$, VEGFR3, PDGFR.

**FABS-NMR screening**

The biochemical functional NMR screening on the proteins kinase AKT-1 and PKA was performed using the 3-FABS (**3-F**luorine **A**toms for **B**iochemical **S**creening) technique developed by C. Dalvit and the NMR group in house (C. Dalvit et al 2003 J. Am. Chem. Soc. 125 (47): 14620).

The method require the use of a $^{19}F$-labelled substrate. The same commercial peptide was used as substrate in both enzymatic reaction: the Tyr just before the phosphorilation site in the AKTide peptide was mutated in a 3-tryfluormethyl-Phe by Bachem.

The AKTide like peptide sequence is reported below:

H- Ala- Arg- Lys- Arg- Glu- Arg- Ala- Phe (3- CF3)- Ser- Phe- Gly- His- His- Ala- OH

The active human Ser-Thr protein kinase AKT-1 Full Length was expressed as GST fusion protein in insect cells and then the GST was cleaved with PreScission protease.

The active cycled AMP-dependent human protein kinase A PKA was expressed as polyHis-tag protein in E.Coli and purified by affinity column.

Reactions were performed in an end point format in eppendorf of 0.5 ml using the buffer solution 50mM Tris, pH 7.5, 5mM $MgCl_2$, 1mM DTT, 8% D2O and 0.001% Triton-X-100; the final volume was of 560 $\mu$l. The substrate and protein concentration were of 30 $\mu$M and 25 nM respectively. The reactions were performed using an ATP concentration of about 9 times its $K_M$: 150 $\mu$M in the test with PKA and 1.5 mM in the AKT-1 experiments. A sample in absence of inhibitor was run as control in order to follow the ongoing of the reaction and it represents the 0% inhibition. Different concentration compound stock solutions in DMSO*d6* were made in order to add the same amount of DMSO*d6* in each sample: usually the compounds stock were of 0.03, 0.2, 2, and 10 mM. 0.56 $\mu$l of these solution was added in each sample in order to reach the final compounds concentration of 30 nM, 200 nM 2 $\mu$M and 10 $\mu$M respectively; 0.56 $\mu$l of DMSO*d6* was added in the control too. The reactions were quenched after about 100/120 minutes with 10 mM of Staurosporine (Sigma code:S4400), transferred into the 5 mm NMR tube (Cortec code: NE-MP5-8) and put onto the Sample Management System (SMS) autosampler for automatic data collection. The spectra were recorded using the Varian Inova 600 MHz NMR spectrometer operating at a $^{19}F$ Larmor frequency of 564 MHz and using a 5mm probe with the inner coil tuned to $^{19}F$ and the outer coil tuned to H. The data were recorded without proton decoupling with a spectral width of 12000 Hz, an acquisition time of 0.8s and a relaxation delay of 2.8s. Chemical shifts are referenced to trifluoroacetic acid.

$IC_{50}$ determination was achieved by measuring the substrate signal as a function of the inhibitor concentration using one of the formulas below:

$$[S_w] = \frac{[S_{w/o}] - [S_{TOT}]}{1 + \left(\frac{[I]}{IC_{50}}\right)^n} + [S_{TOT}]$$

where [$S_w$] and [$S_{w/o}$] are given by the integrals of the substrate signal in the presence and absence of the inhibitor, respectively. [$S_{TOT}$] is the substrate concentration used for the experiments and it represents an internal reference, [I] is the concentration of the inhibitor, $IC_{50}$ the concentration of the inhibitor at which 50% inhibition is observed and *n* is the cooperativity factor (known also as Hill slope) . In the absence of allosteric effects (i.e. $n = 1$) a meaningful value for $IC_{50}$ is derived with a single (or very few 2- 3) experimental point . This is possible because the values for both plateaus are known. These are $S_{w/o}$ for the 0% inhibition and $S_{TOT}$ for the 100% inhibition. $IC_{50}$ data were analyzed with non-linear least squares methods in the Origin 5.0 software package.

Biochemical, NMR and cell- based S6 phosphorylation in MCF7- cell assay data for representative compounds are reported in the following Table 1.

Table 1.

| COMPOUND | AKT1 $IC_{50}$ ($\mu$M) Biochemical assay | AKT2 $IC_{50}$ ($\mu$M) Biochemical assay | AKT3 $IC_{50}$ ($\mu$M) Biochem ical assay | AKT1 $IC_{50}$ ($\mu$M) FABS NMR assay | PKA$\alpha$ $IC_{50}$ ($\mu$M) FABS NMR assay | S6 phosphorylation (Ser235/236) in MCF7-cell $IC_{50}$ ($\mu$M) |
|---|---|---|---|---|---|---|
| 1 | 0.017 | 0.092 | 0.019 | 0.062 | 0.025 | 1.35 |
| 10 | 0.149 | 0.786 | 0.108 | 0.062 | 1.9 | 16.00 |

The compounds of the present invention can be administered either as single agents or, alternatively, in combination with known anticancer treatments such as radiation therapy or chemotherapy regimen in combination with cytostatic or cytotoxic agents, antibiotic-type agents, alkylating agents, antimetabolite agents, hormonal agents, antihormonal agents such as antiestrogens, antiandrogens and aromatase inhibitors, immunological agents, interferon-type agents, cyclooxygenase inhibitors (e.g. COX-2 inhibitors), matrixmetalloprotease inhibitors, telomerase inhibitors, kinase inhibitors, anti-growth factor receptor agents, anti-HER agents, anti-EGFR agents, anti-angiogenesis agents (e.g. angiogenesis inhibitors), farnesyl transferase inhibitors, ras-raf signal transduction pathway inhibitors, cell cycle inhibitors, other cdks inhibitors, tubulin binding agents, topoisomerase I inhibitors, topoisomerase II inhibitors, agents that target microtubules, platin-based agents, DNA damaging or intercalating agents, inhibitors of kinesins, therapeutic monoclonal antibodies, inhibitors of mTOR, histone deacetylase inhibitors, and inhibitors of hypoxic response.

If formulated as a fixed dose, such combination products employ the compounds of this invention within the dosage range described below and the other pharmaceutically active agent within the approved dosage range.

Compounds of formula (I) may be used sequentially with known anticancer agents when a combination formulation is inappropriate.

The compounds of formula (I) of the present invention, suitable for administration to a mammal, e.g., to humans, can be administered by the usual routes and the dosage level depends upon the age, weight, conditions of the patient and administration route.

For example, a suitable dosage adopted for oral administration of a compound of formula (I) may range from about 10 to about 500 mg per dose, from 1 to 5 times daily. The compounds of the invention can be administered in a variety of dosage forms, e.g., orally, in the form tablets, capsules, sugar or film coated tablets, liquid solutions or suspensions; rectally in the form suppositories; parenterally, e.g., intramuscularly, or through intravenous and/or intrathecal and/or intraspinal injection or infusion.

The present invention also includes pharmaceutical compositions comprising a compound of formula (I) or a pharmaceutically acceptable salt thereof in association with a pharmaceutically acceptable excipient, which may be a carrier or a diluent.

The pharmaceutical compositions containing the compounds of the invention are usually prepared following conventional methods and are administered in a suitable pharmaceutical form. For example, the solid oral forms may contain, together with the active compound, diluents, e.g., lactose, dextrose saccharose, sucrose, cellulose, corn starch or potato starch; lubricants, e.g., silica, talc, stearic acid, magnesium or calcium stearate, and/or polyethylene glycols; binding agents, e.g., starches, arabic gum, gelatine methylcellulose, carboxymethylcellulose or polyvinyl pyrrolidone; disintegrating agents, e.g., starch, alginic acid, alginates or sodium starch glycolate; effervescing mixtures; dyestuffs; sweeteners; wetting agents such as lecithin, polysorbates, laurylsulphates; and, in general, non-toxic and pharmacologically inactive substances used in pharmaceutical formulations. These pharmaceutical preparations may be manufactured in known manner, for example, by means of mixing, granulating, tabletting, sugar-coating, or film-coating processes.

The liquid dispersions for oral administration may be, e.g., syrups, emulsions and suspensions. As an example, the syrups may contain, as carrier, saccharose or saccharose with glycerine and/or mannitol and sorbitol.

The suspensions and the emulsions may contain, as examples of carriers, natural gum, agar, sodium alginate, pectin, methylcellulose, carboxymethylcellulose, or polyvinyl alcohol. The suspension or solutions for intramuscular injections may contain, together with the active compound, a pharmaceutically acceptable carrier, e.g., sterile water, olive oil, ethyl oleate, glycols, e.g., propylene glycol and, if desired, a suitable amount of lidocaine hydrochloride.
The solutions for intravenous injections or infusions may contain, as a carrier, sterile water or preferably they may be in the form of sterile, aqueous, isotonic, saline solutions or they may contain propylene glycol as a carrier.
The suppositories may contain, together with the active compound, a pharmaceutically acceptable carrier, e.g., cocoa butter, polyethylene glycol, a polyoxyethylene sorbitan fatty acid ester surfactant or lecithin.
With the aim of better illustrating the present invention, without posing any limitation to it, the following examples are now given.

## EXAMPLES

For a reference to any specific compound of formula (I) of the invention, optionally in the form of a pharmaceutically acceptable salt, see the experimental section and claims. Referring to the examples that follow, compounds of the present invention were synthesized using the methods described herein, or other methods, which are well known in the art.
The short forms and abbreviations used herein have the following meaning:

| | |
|---|---|
| g (grams) | mg (milligrams) |
| ml (milliliters) | mM (millimolar) |
| μM (millimo lar) | mmo l (millimoles) |
| h (hours) | MHz (Mega-Hertz) |
| mm (millimetres) | Hz (Hertz) |
| M (molar) | min (minutes) |
| mol (moles) | TLC (thin layer chromatography) |
| r.t. (room temperature) | TEA (triethylamine) |
| TFA (trifluoroacetic acid) | DMF (N,N-dimethyl formamide) |
| DIPEA (N,N-diisopropyl-N-ethylamine) | DCM (dichloromethane) |
| THF ( tetrahydrofuran) | tBuOK ( potassium terbuthylate) |
| MeOH (Methanol) | Hex (hexane) |
| EtSiH (triethylsilane) | TSE (*p*-toluensolfonylethyl) |
| PMB (*p*-methoxy benzyl) | DMSO (dimethylsulfoxide) |
| BOC (tert-butyloxycarbonyl) | bs (broad singlet) |
| NaH = sodium hydride, 60% in mineral oil | Ac (acetyl) |
| $Ac_2O$ acetic anhydride | ESI = electrospray ionization |
| TBTU (2-(1H-benzotriazol-1-yl)-1,1,3,3-tetramethyluroniumtetrafluoroborate | |
| RP-HPLC (reverse phase high performance liquid chromatography) | |

### General purification and analytical methods

The compounds of formula (I) were prepared according to the previously described synthetic process and individual examples are detailed therein.
The examples are provided for the purpose of illustration only and are not intended as limiting the scope of the invention.
The compounds were named using ACDLABS 9.0.
As used herein the symbols and conventions used in the processes and examples are consistent with those used in the contemporary scientific literature, for example, the *Journal of the American Chemical Society* or the *Journal of Biological Chemistry*.
Unless otherwise noted, all materials were obtained from commercial suppliers, of the best grade and used without further purification. Anhydrous solvent such as DMF, THF, $CH_2Cl_2$ and toluene were obtained from the Aldrich Chemical Company. All reactions involving air- or moisture-sensitive compounds were performed under nitrogen atmosphere. Flash chromatography was performed using Aldrich Chemical Company silica gel (200-400 mesh, 60A).
Flash Chromatography was performed on silica gel (Merck grade 9395, 60A) and TLC were performed on Merck silica gel (5 x 10 mm) plates coated with 250 μm layer and fluorescent indicator. Components were visualized

by UV light (λ=254 nm ).

HPLC was performed on Waters X Terra RP 18 (4,6 x 50 mm, 3.5 μm) column using a Waters 2790 HPLC system equipped with a 996 Waters PDA detector and Micromass mod. ZQ single quadrupole mass spectrometer, equipped with an electrospray (ESI) ion source.

Mobile phase A was ammonium acetate 5 mM buffer (pH 5.5 with acetic acid-acetonitrile 95:5), and Mobile phase B was water-acetonitrile (5:95). Gradient from 10 to 90% B in 8 minutes, hold 90% B 2 minutes. UV detection at 220 nm and 254 nm. Flow rate 1 mL/min. Injection volume 10 microL. Full scan, mass range from 100 to 800 amu. Capillary voltage was 2.5 KV; source temperature was 120°C; cone was 10 V. Retention times (HPLC r.t.) are given in minutes at 220 nm or at 254 nm. Mass are given as m/z ratio.

When necessary, compounds were purified by preparative HPLC on a Waters Symmetry C18 (19 x 50 mm, 5 um) column or on a Waters X Terra RP 18 (30 x 150 mm, 5 μm) column using a Waters preparative HPLC 600 equipped with a 996 Waters PDA detector and a Micromass mod. ZMD single quadrupole mass spectrometer, electron spray ionization, positive mode. Mobile phase A was water-0.01% trifluoroacetic acid, and mobile phase B was acetonitrile. Gradient from 10 to 90% B in 8 min, hold 90% B 2 min. Flow rate 20 mL/min. In alternative, mobile phase A was water-0.1 % $NH_3$, and mobile phase B was acetonitrile. Gradient from 10 to 100% B in 8 min, hold 100% B 2 min. Flow rate 20 mL/min.

$^1$H- NMR spectrometry was performed on a Mercury VX 400 operating at 400.45 MHz equipped with a 5 mm double resonance probe [1H (15N- 31P) ID_ PFG Varian] . $^1$H- NMR were recorded in DMSO- d6 at 400 MHz, chemical shifts are expressed in ppm (δ) .

The physical data given for the compounds exemplified is consistent with the assigned structure of those compounds.

__Example 1__

**Preparation of (2*E*)-3-[1-(4-methoxybenzyl)-1*H*-pyrazolo[3,4-*b*]pyridin-3-yl]acrylic acid [(VIII), R2= H]**

__Step1.__ **3-Iodo-1H-pyrazolo[3,4-b]pyridine [(IV)]**

The synthesis of this intermediate, Registry Number 117007- 52- 0, is performed as described in Tetrahedron Lett., 41 (2000) 4363, starting from Reg. N. 18417- 64- 6, described in Can. J.Chem., 66, (1988), 420- 428.
HRMS (ESI) : 245.9527 ($C_6$ $H_4$ I $N_3$, M+1; calc 245.9523)
$^1$H- NMR (400 MHz), δ (ppm, DMSO- $d_6$) : 14.08 (s, 1H), 8.59 (dd, J=4.51, 1.59 Hz, 1H), 7.49 (m, 1H), 7.28 (dd, J=8.05, 4.51 Hz, 1H) .

__Step 2.__ **3-Iodo-1-(4-methoxy-benzyl)-1H-pyrazolo[3,4-b]pyridine [(V), R7 = 4-methoxy-benzyl]**

3- Iodo- 1H- pyrazolo [3, 4- b] pyridine (2.5 g, 10.2 mmol) in about 30 ml of dry DMF, is reacted with *p*- methoxy-benzylchloride (4.15 ml, 30.6 mmol) and finely grinded potassium hydroxide (30.6 mmol, 1.71 g) . The reaction is heated at 70° C for 3 h, cooled down to room temperature, poured into a separating funnel containing water/ Ethyl Acetate and extracted several times. The organic phase is dried over $Na_2SO_4$, filtered and evaporated down. The purification by silica chromatography (Hex/ Ethyl Acetate 1/1) gives 3.35 g of clean product (90 % yield) .
HRMS (ESI) : 366.0099 ($C_{14}$ $H_{12}$ I $N_3$ O, M+1; calc 366.0098)
$^1$H- NMR (400 MHz), δ (ppm, DMSO- $d_6$) : 8, 66 (dd, J= 4.5, 1.4 Hz, 1H), 7.96 (dd, J=8.5, 1.4 Hz, 1H), 7.33 (dd, J= 8.05, 4.51 Hz , 1H) 7.28- 7.23 (m, 2H), 6.91- 6.86 (m, 2H), 5.62 (s, 2H), 3.72 (s, 3H) .

__Step 3.__ **(E)-3-[1-(4-methoxy-benzyl)-1H-pyrazolo[3,4-b] pyridin-3-yl]-acrylic acid ethyl ester [(VI), R7 = 4-methoxy-benzyl, R2= H]**

3- Iodo- 1- (4- methoxy- benzyl)- 1H- pyrazolo [3, 4- b] pyridine (2.88 mmol, 1.05g) is suspended under argon in dry and degassed acetonitrile; ethyl acrilate (2.45 ml, 23.0 mmol), Pd $(OAc)_2$ (130 mg, 0.576 mmol), P $(oTol)_3$ (350 mg, 1.152 mmol) and TEA (2.02 ml, 14.4 mmol) are added and the mixture is heated at 80° C for 2.5 hours. The reaction is cooled to r.t., evaporated to small volume, diluted with water and ethyl acetate. The organic phase is dried over $Na_2SO_4$, filtered and evaporated the crude is purified by silica chromatography (Hexane/ Ethyl acetate- 7/3) to give 840 mg of clean compound (86 % yield) .
HRMS (ESI) : 338.151 ($C_{19}$ $H_{19}$ $N_3$ $O_3$, M+1; calc 338.1499)
$^1$H- NMR (400 MHz), δ (ppm, DMSO- $d_6$) : 8.70 (dd, J=8.17, 1.46 Hz, 1H), 8.68 (dd, J=4.51, 1.46 Hz, 1H), 7.83 (d, J=16.34 Hz, 1H), 7.38 (dd, J=8.11 Hz, J= 4.57 Hz, 1H), 7.32- 7, 26 (m, 2 H), 6.91- 6.86 (m, 2H), 6.84 (d, J=16.22 Hz, 1 H), 5.67 (s, 2H), 4.24 (q, J=7.07 Hz, 2 H), 3.71 (s, 3 H), 1.30 (t, =7.07 Hz, 3H) .

**Step 4. (2*E*)-3-[1-(4-methoxybenzyl)-1*H*-pyrazolo[3,4-*b*]pyridin-3-yl]acrylic acid [(VII), R7 = 4-methoxy-benzyl, R2= H]**

(E)- 3- [1- (4- methoxy- benzyl)- 1H- pyrazolo [3, 4- b] pyridin- 3- yl]- acrylic acid ethyl ester (840 mg, 2.49 mmol) are suspended in about 40 ml of ethanol, then 2 ml of NaOH 5M are added and the mixture is heated at 60°C. The hydrolysis is complete after 3 hours and the reaction is dried under vacuum. The residue, dissolved in water, is acidified with glacial acetic acid and extracted with ethyl acetate. The organic phase is dried over $Na_2SO_4$, filtered and evaporated down to give 636 mg of clean product (82 % yield) .
HRMS (ESI) : 310.1201 ($C_{17}H_{15}N_3O_3$, M+1; calc 310.1186)
$^1$H- NMR (400 MHz), δ (ppm, DMSO- $d_6$) : 8.66 (dd, J=4.51, 1.46 Hz, 1H) , 8.64 (dd, J=8.1, 1.4 Hz, 1H) 7.75 (d, J= 16.2 Hz, 1H), 7.36 (dd, J=8.0, 4.5 Hz, 1H), 7.29- 7.23 (m, 2H), 6.90- 6.84 (m, 2H), 6.75 (d, J=16.28 Hz, 1H), 5.65 (s, 2H), 3.70 (s, 3H) .

**Step 5. *tert*-butyl [(2*S*)-2-({(2*E*)-3-[1-(4-methoxybenzyl)-1*H*-pyrazolo[3,4-*b*]pyridin-3-yl]prop-2-enoyl}amino)-3-phenylpropyl]carbamate [(VIII), R7 = 4-methoxybenzyl, R2= H, R1' = benzyl, m = 1, R3= tert-butyl carbamate, R4= H]**

(2*E*)- 3- [1- (4- methoxybenzyl)- 1H- pyrazolo [3, 4- b] pyridin- 3- yl] acrylic acid (0.4 mmol) is reacted at room temperature with *tert*- butyl [(2*S*)- 2- amino- 3- phenylpropyl] carbamate (0.43 mmol) in dry DMF and in the presence of TBTU (0.45 mmoles) and DIPEA (0.6 mmol) . The mixture is let stir overnight, diluted with ethyl acetate and washed with water and 5% $NaHCO_3$. The organic phase is dried over $Na_2SO_4$, filtered and evaporated down. The yellowish oil was purified on silica gel (Hex/ Ethyl Acetate- 1/1) to give 150 mg of clean compound.
HRMS (ESI) : 542.2787 ($C_{31}H_{35}N_5O_4$, M+1; calc 542.2762)
$^1$H- NMR (400 MHz), δ (ppm, DMSO- $d_6$) : 8.66 (dd, J=4.51, 1.46 Hz, 1H), 8.51 (dd, J=8.05, 1.46 Hz, 1H), 8.02 (d, J= 8.41 Hz, 1H), 7.53 (d, J= 16.10, 1H), 7.39 (dd, J= 8.05, 4.51 Hz, 1H), 7.31- 7.15 (m, 7H), 6.98 (d, J= 16.10 Hz, 1H), 6.88 (m, 3H), 5.64 (s, 1H), 4.21- 4.08 (m, 1H), 3.71 (s, 3H), 3.17- 3.03 (m, 2H), 2.84 (dd, J=13.96, 5.24 Hz, 1H), 2.65- 2.37 (m, 1H), 1.37 (s, 9H) .

**Step 6. (*E*)-N-((S)-2-amino-1benzyl-ethyl)-3-(1*H*-pyrazolo[3,4-*b*] pyridin-3-yl)acrylamide tris(trifluoroacetate) [R2= H, R1' = benzyl, m = 1, R3= R4= H]**

**Cpd. 1**

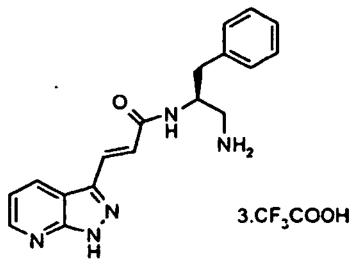

3.$CF_3COOH$

*tert*- butyl[(2*S*)- 2- ( {(2*E*)- 3- [1- (4- methoxybenzyl)- 1*H*- pyrazolo [3, 4- *b*] pyridin- 3- yl] prop- 2- enoyl} amino)- 3- phenylpropyl] carbamate is afterward dissolved in TFA in the presence of 200µl of $Et_3SiH$ and is reacted overnight @65°C. The crude compound is purified by RP- HPLC and recovered as trifluoroacetate salt.
HRMS (ESI) : 322.1677 ($C_{18}H_{19}N_5O$ M+1; calc 322.1662)
$^1$H- NMR (400 MHz), δ (ppm, DMSO- $d_6$) : 14.03 (s, 1H), 8.61 (d, J=4.3 Hz 1H), 8.48 (d, J= 7.9 Hz, 1H), 8.32 (d, J= 8.2Hz, 1H), 7.89 (bs, 2H), 7.62 (d, J=16.1 Hz, 1H), 7.42- 7.19 (m, 5H), 6, 99 (d, J=16.1Hz, 1H), 4.35 (m, 1H), 3.11- 2.77 (m, 4H) .

Operating in an analogues way, the following compounds were prepared:

**(2*E*)-3-(1H-pyrazolo[3,4-b]pyridin-3-yl)acrylamide tris(trifluoroacetate) [R2= H, R1= H]**

**Cpd. 2**

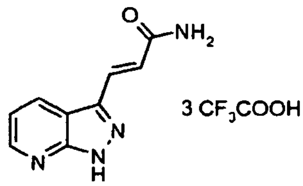

ESI (+) MS for $C_9H_8N_4O$ m/z 189 ($MH^+$)
$^1$H- NMR (400 MHz), δ (ppm, DMSO- *d*$_6$) : 13.98 (bs, 1H), 8.60 (dd, J=4.5, 1.5 Hz, 1H) 8.50 (dd, J= 8.1, 1.5 Hz, 1H) 7.62 (d, J=16.1 Hz, 1H) 7.60 (s, 1H) 7.34 (dd, J= 8.1, 4.5 Hz, 1H), 7.20 (s, 1H), 7.02 (d, J=16.1 Hz, 1H) .

**(2*E*)-N-(3,4-difluorobenzyl)-3-(1H-pyrazolo[3,4-b]pyridin-3-yl)acrylamide [R2= H, R1= 3,4-difluorobenzyl]**

**Cpd. 3**

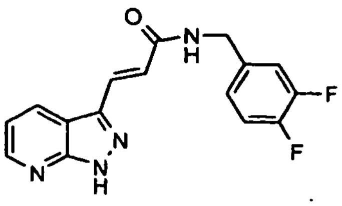

HRMS (ESI) : 315.104 ($C_{24}H_{20}F_2N_4O$, M+1; calc 315.1052)
$^1$H- NMR (400 MHz), δ (ppm, DMSO- *d*$_6$) : 14.00 (s, 1H), 8.70 (t, J =5.7 Hz, 1H), 8.60 (dd, J= 4.5, 1.4 Hz, 1H), 8.50 (dd, J= 8.0, 0.7 Hz, 1H) 7.67 (d, J=16.10 Hz, 1H), 7.48- 7.33 (m, 2H) 7.34 (dd, J= 8.0, 4.5, 1H), 7.23- 7.16 (m, 1H), 7.10 (d, J=16.10, 1H), 4.44 (d, J=5.7 Hz, 2H) .

**(2*E*)-*N*-[(1*S*)-1-phenyl-2-pyrrolidin-1-ylethyl]-3-(1*H*-pyrazolo[3,4-*b*]pyridin-3-yl)acrylamide [ R2= H, R1 = (1*S*)-1-phenyl-2-pyrrolidin-1-ylethyl]**

**Cpd. 4**

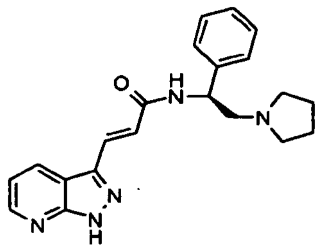

HRMS (ESI) : 362.199 ($C_{21}H_{23}N_5O$, M+1; calc 362.1975)
$^1$H- NMR (400 MHz), δ (ppm, DMSO- *d*$_6$) : 13.99 (s, 1H), 8.64- 8.58 (bs, 1H), 8.61 (dd, J= 4.5, 1.4 Hz, 1H), 8.54 (dd, J = 8.0, 1.4 Hz, 1H), 7.60 (d, J=16.1 Hz, 1H), 7.44- 7.32 (m, 5H), 7.31- 7.24 (m, 1H), 7.17 (d, J=16.1 Hz, 1H), 5.13 (bs, 1H), 2.92 (bs, 2H), 2.52 (bs, 4H), 1.71 (bs, 4H) .

**(2*E*)-N-[(1S)-3-amino-1-benzylpropyl]-3-(1H-pyrazolo[3,4-b]pyridin-3-yl)acrylamide [ R2= H, R1' = benzyl, m= 2, R3= R4= H]**

**Cpd. 5**

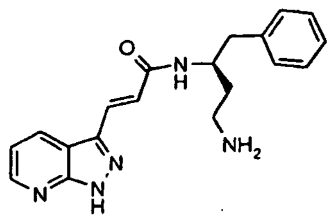

HRMS (ESI) : 336.1827 ($C_{19}$ $H_{21}$ $N_5$ O, M+1; calc 336.1819)
$^1$H- NMR (400 MHz), δ (ppm, DMSO- *d*$_6$) : 8.60 (dd, J=4.5, 1.4 Hz, 1H), 8.48 (dd, J= 8.1, 1, 4 Hz, 1H), 8.06 (d, J=8.4 Hz, 1H), 7.57 (d, J=15.9 Hz, 1H), 7.34 (dd, J= 8.0, 4.5 Hz , 1H), 7.33- 7.15 (m, 5H), 7.02 (d, J=16.1, 1H), 4.27- 4.14 (m, 1H), 2.80 (d, J = 6.83 Hz. 2H), 2.69- 2.53 (m, 2H), 1.69- 1.43 (m, 2H) .

**(2E)-N-[(1S)-2-amino-1-(thien-2-ylmethyl)ethyl]-3-(1H-pyrazo;o[3,4-b]pyridin-3-yl)acrylamide [ R2= H, R1' = thien-2-ylmethyl, m =1, R3= R4= H]**

**Cpd. 6**

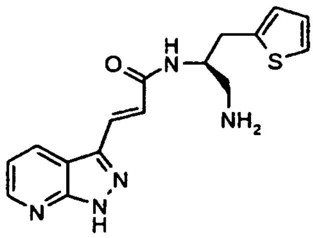

HRMS (ESI): 328.1234 ($C_{16}$ $H_{17}$ $N_5$ O S, M+1; calc 328.1227)
1H-NMR (400 MHz), δ (ppm, DMSO-*d*$_6$): 8.60 (dd, J=4.5, 1.4 Hz, 1 H), 8.51 (dd, J = 8.1, 1.5 Hz, 1H), 8.11 (d, J=8.0 Hz, 1H), 7.60 (d, J= 16.1 Hz, 1H), 7.34 (dd, J= 4.5, 8.1 Hz, 1H), 7.33 (d, J =4.8 Hz, 1H) 7.08 (d, J=16.1 Hz), 6.95 (dd, J=5.1, 3.4 Hz, 1H), 6.92 (dd, J=3.2, 0.9 Hz, 1H), 4.09 (bs, 1H), 3.17 (dd, J=14.6, 5.7 Hz, 1H) 2.97 (dd, J = 5.7, 14.8 Hz 1H), 2.72-2.62 (m, 2H).

**(2*E*)-N-[(1S)-2-morpholin-4-yl-1-phenylethyl]-3-(1H-pyrazolo [3,4-b]pyridin-3-yl)acrylamide [ R2= H, R1 = (1S)-2-morpholin-4-yl-1-phenylethyl]**

**Cpd. 7**

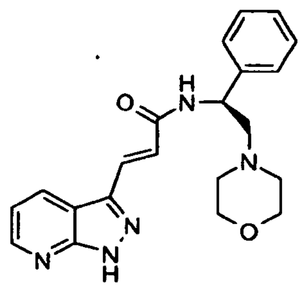

HRMS (ESI) : 378.1939 ($C_{21}$ $H_{23}$ $N_5$ $O_2$, M+1; calc 378.1924)
$^1$H- NMR (400 MHz), δ (ppm, DMSO- $d_6$) : 13.99 (s, 1H), 8.61 (dd, J= 4.5, 1.4 Hz, 1H), 8.5 (d, J= 8.05 Hz, 1H), 7.61 (d, J=16.1 Hz, 1H), 7.45- 7.32 (m, 5H), 7.35 (dd, J=8.0, 4.5 Hz, 1H) 7.16 (d, J= 16.1 Hz, 1H), 5.19 (bs, 1H), 3.56 (bs, 4H), 2.77- 2.66 (bs, 2H), 2.62- 2.37 (m, 4H) .

***N*α-[(2*E*)-3-(1*H*-pyrazolo[3,4-*b*]pyridin-3-yl)prop-2-enoyl]-L-phenylalaninamide [ R2= H, R1' = benzyl, m = 1, R3= R4= H]**

**Cpd. 8**

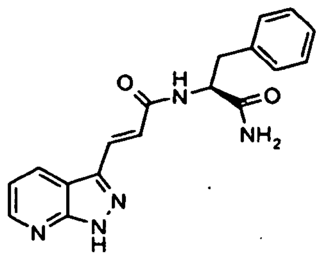

HRMS (ESI) : 336.1461 ($C_{18}$ $H_{17}$ $N_5$ $O_2$, M+1; calc 336.1455)
$^1$H- NMR (400 MHz), δ (ppm, DMSO- $d_6$) : 13.96 (s, 1H), 8.60 (dd, J= 4.5, 1.4 Hz, 1H), 8.55 (dd, J=8.0, 1.4 Hz, 1H), 8.35 (d, J=8.4 Hz, 1H), 7.58 (bs, 1H), 7.55 (d, J=16.2 Hz, 1H), 7.34 (dd, J = 8.1, 4.5 Hz, 1H), 7.31- 7.25 (m, 4H), 7.23- 7.16 (m, 1H), 7.16 (d, J= 16.1 Hz, 1H), 7.11 (bs, 2H), 4.67 (m, 1H), 3.09 (dd, J=13.9, 5.0 Hz, 1H) 2.86 (dd, J= 13.7, 9.0 Hz, 1H) .

**(2*E*)-*N*-[(1*S*)-2-(4-methylpiperazin-1-yl)-1-phenylethyl]-3-(1*H*-pyrazolo[3,4-*b*]pyridin-3-yl)acrylamide [R2= H, R1 = (1*S*)-2-(4-methylpiperazin-1-yl)]**

**Cpd. 9**

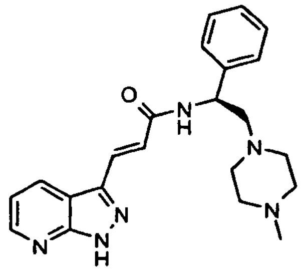

HRMS (ESI) : 391.2255 ($C_{22}H_{26}N_6O$, M+1; calc 391.2241)
$^1$H- NMR (400 MHz), δ (ppm, DMSO- $d_6$) : 13.99 (s, 1H), 8.61 (dd, J=4.5, 1.4 Hz, 1H), 8.57 (d, J= 8.2 Hz, 1H), 8.53 (dd, J= 8.0, 1.4 Hz, 1H), 7.60 (d, J= 16.1 Hz, 1H), 7.41- 7.22 (m, 6H), 7.16 (d, J=16.10 Hz, 1H), 5.16 (m, 1H), 2.71 (dd J = 9.75, 12.6 Hz, 2H), 2.55- 2.29 ( m, 8H), 2.18 (bs, 3H) .

## Example 2

**Preparation of (2*E*)-2-(5-bromo-1*H*-indol-3-yl)-3-(1-{2-[(4-methylphenyl)sulfonyl] ethyl}-1*H*-pyrazolo[3,4-*b*]pyridin-3-yl)acrylic acid [(VIII) R2= -1*H*-indol-3-yl, R5 = methyl, R6 = bromine, R7 = [(4-methylphenyl)sulfonyl]ethyl)].**

**Step1. 3-iodo-1-{2-[(4-methylphenyl)sulfonyl]ethyl}-1*H*-pyrazolo[3,4-*b*]pyridine [(V), (R7 = [(4-methylphenyl)sulfonyl] ethyl)]**

2, 2 g of 3- iodo- 1H- pyrazolo [3, 4- b] pyridine are dissolved in 40ml of dry DMF and reacted with 1.797g of 1- ethenesulfonyl- 4- methyl- benzene (prepared according to Synth. Comm., (2000) 30 (16), 2897- 2902) in the presence of 2.5 g $K_2CO_3$ at 65°C. The reaction, completed in three hours, is worked up in a separating funnel with water and ethyl acetate. The organic phase is then washed with brine, dried over $Na_2SO_4$, and evaporated down to give 3.8 g of white product. Yield: quantitative
HRMS (ESI) : 427.9931 ($C_{15}H_{14}IN_3O_2S$, M+1; calc 427.9924)
$^1$H- NMR (400 MHz), δ (ppm, DMSO- d6) : 8.60 (dd, J=4.5, 1.4 Hz, 1H), 7.80 (dd, J=1.4, 8.05, 1H), 7.41 (m, 2H), 7.26 (dd, J=8.05, 4.5 Hz, 1H), 7.10 (m, 2H), 4.79 (m, 2H), 4.06 (m, 2H), 2.29 (s, 3H) .

**Step2. 1-{2-[(4-methylphenyl)sulfonyl]ethyl}-1*H*-pyrazolo[3,4-*b*]pyridine-3-carboxylic acid [(IX), (R7 = [(4-methylphenyl)sulfonyl] ethyl)]**

2 g of 3- iodo- 1- {2- [(4- methylphenyl) sulfonyl] ethyl}- 1H- pyrazolo [3, 4- b] pyridine, dissolved in 25 ml of anhydrous DMF and in the presence of 430 mg of $Pd_2(dba)_3$, are reacted with 2.2 g of lithium formiate, 4.8 ml of DIPEA, 987 mg of LiCl, 2.66 ml of acetic anhydride, which are added in the end. *(*Org, Lett. 5, (2003) , 23, 4269- 4272*)* .
The mixture is stirred at 80 °C for 5 hours.
The reaction, filtered through a pad of celite, is evaporated under vacuum to half volume. The remaining DMF is poured into a separating funnel containing 200 ml of water, which is extracted several times with ethyl acetate. The product is contained in the water phase, which is afterwards acidified with acetic acid and is extracted several times with ethyl acetate. This organic phase, dried, filtered and evaporated, gives 1,15 g of white powder (about 70% yield) suitably pure to be directly used in the next step.
HRMS (ESI): 346.0863 ($C_{16}H_{15}N_3O_4S$, M+1; calc 346.0856)
1H-NMR (400 MHz), δ (ppm, DMSO-d6): 13.33 (bs, 1H), 8.62 (dd, J=4.51, 1.59 Hz, 1H), 8.34 (dd, J=8.17, 1.59 Hz, 1H), 7.48 (d, J=8.41 Hz, 2H), 7.38 (dd, J=8.11, 4.45 Hz, 1 H), 7.13 (d, J=7.93, 2H), 4.85 (t, J=6.28 Hz, 2H), 4.08 (t, J = 6.28 Hz, 2H).

**Step3. *N*-methoxy-*N*-methyl-1-{2-[(4-methylphenyl)sulfonyl]ethyl}-1*H*-pyrazolo[3,4-*b*]pyridine-3-carboxamide [(X), (R7 = [(4-methylphenyl)sulfonyl] ethyl)]**

To a solution of 1g 1- {2- [(4- methylphenyl) sulfonyl] ethyl}- 1H- pyrazolo [3, 4- b] pyridine- 3- carboxylic acid in 50 ml of DMF was added 338 mg of N, O- dimethyl hydroxylamine hydrochloride followed by 1.11 g of TBTU and about 1.5 ml of DIPEA.
The reaction, complete in 3 hours, is worked up with water, ethyl acetate and 5% $NaHCO_3$. The organic phase is dried, filtered and evaporated to give 1.05 g of Weinreb amide (95 % yield) .
HRMS (ESI) : 389.1287 ($C_{18}H_{20}N_4O_4S$, M+1; calc 389.1278)
$^1$H- NMR (400 MHz), δ (ppm, DMSO- d6) : 8.61 (dd, J=4.51, 1.59 Hz, 1 H), 8.32 (dd, J=8.17, 1.59 Hz, 1 H), 7.49 (d, J=8.17 Hz, 2 H), 7.34 (dd, J=8.05, 4.45 Hz, 1 H), 7.13 (d, J=7.93 Hz, 2 H), 4.84 (t, J=6.00 Hz, 2 H), 4.09 (t, J=6.16 Hz, 2 H) 3.78 (s, 3H), 3.44 (s, 3H), 2.26 (s, 3H) .

**Step 4. 1-{2-[(4-methylphenyl)sulfonyl] ethyl}-1*H*-pyrazolo[3,4-*b*]pyridine-3-carbaldehyde [(XI), (R7 = -(4-methylphenyl)sulfonyl ethyl]**

To a solution of *N*- methoxy- *N*- methyl- 1- {2- [(4- methylphenyl) sulfonyl] ethyl}- 1*H*- pyrazolo [3, 4- *b*] pyridine- 3- carboxamide (1.390g, 3, 58 mmol) in THF (30 ml) at- 78 °C was added $LiAlH_4$ (3, 6 ml, 1 M in THF) . The mixture was stirred for 2.5 hours and cautiously quenched with methanol, keeping the low temperature for a further half an hour

and then warming up the flask to room temperature. The reaction was concentrated to remove THF and the residue was partitioned between water and ethyl acetate, acidifying with citric acid. The combined organic phases were dried, filtered and evaporated to afford 1, 165g of white product in a quantitative yield.
HRMS (ESI) : 330.0916 ($C_{16} H_{15}N_3 O_3$ S, M+1; calc 330.0907)
$^1$H- NMR (400 MHz), δ (ppm, DMSO- d6) : 9.97 (s, 1 H), 8.68 (dd, J=4.45, 1.52 Hz, 1 H), 8.40 (dd, J=8.05, 1.34 Hz, 1 H), 7.45 (d, J=8.17 Hz, 2 H), 7.45 (dd, J=8.05, 4.30 Hz, 1 H), 7.09 (d, J=8.05 Hz, 2 H), 4.92 (t, J=6.10 Hz, 2 H), 4.16 (t, J=6.10 Hz, 2 H), 2.24 (s, 3 H) .

**Step 5. (2*E*)-2-(5-bromo-1*H*-indol-3-yl)-3-(1-{2-[(4-methylphenyl)sulfonyl]ethyl}-1*H*-pyrazolo[3,4-*b*]pyridin-3-yl) acrylic acid [(VII), R2= 5-bromo-1*H*-indol-3-yl), R7 = [(4-methylphenyl)sulfonyl] ethyl]**

A solution of 5- bromo- 1- methyl- 1H- indol- 3- yl acetic acid (2 mmol) and 1- {2- [(4- methylphenyl) sulfonyl] ethyl}- 1H- pyrazolo [3, 4- b] pyridine- 3- carbaldehyde (659 mg, 2 mmol) in acetic anhydride (3 ml, reagent and solvent) and TEA (1.12 ml, 8 mmol) is strirred at 80 °C overnight. The mixture is cooled and shaken for 4- 5 hours in the presence of 2 ml of NaOH 4N, added to decompose the excess of anhydride. After the addition of $NaH_2PO_4$ 20 % until pH=5, the reaction is worked up with water and ethyl acetate. The organic phase is dried over $Na_2SO_4$, filtered and evaporated to give a brownish oil, which contains the product and is submitted to the next step without purification.
HRMS (ESI) : 579.0670 ($C_{27} H_{23}$ Br $N_4 O_4$ S, M+1; calc 579.0696)
$^1$H- NMR (400 MHz), δ (ppm, DMSO- d6) : 12.80 (bs, 1H), 8.39 (dd, J= 4.39, 1.59 Hz, 1H), 7.78 (s, 1H), 7.44 (d, J= 6.46 Hz, 2H), 7.42 (d, J=6.83, 1H), 7.16 (dd, J=8.78, 1.95, 1H), 7.14 (d, J=7.80 Hz, 2H), 7.11 (d, J=1.71 Hz, 1H), 6.96 (dd, J=8.17, 1.46, 1H), 6.89 (dd, J=8.17, 4.39 Hz, 1H), 4.69 (t, J=6.58 Hz, 2H), 3.79 (t, J= 6.58 Hz, 2H), 3.77 (s, 3H), 2.28 (s, 3H) .

**Step 6. (2*E*)-2-(5-bromo-1-methyl-1*H*-indol-3-yl)-3-(1-{2-[(4-methylphenyl)sulfonyl] ethyl}-1*H*-pyrazolo[3,4-*b*]pyridin-3-yl)-*N*-[(1*S*)-2-(4-methylpiperazin-1-yl)-1-phenylethyl] acrylamide [(VIII), R2= 5-bromo-1-methyl-1*H*-indol-3-yl, R7 = [(4-methylphenyl)sulfonyl] ethyl, R1 = (1*S*)-2-(4-methylpiperazin-1-yl)-1-phenylethyl]**

A portion of the crude containing the acid (0.2 mmol) is diluted in dry DMF and reacted overnight at room temperature with 2- (4- methylpiperazin- 1- yl)- 1- phenylethanamine trihydrochloride (0.24 mmol) in the presence of TBTU (0.24 mmol) and DIPEA (1.2 mmol) .

The mixture is worked up with ethyl acetate, water and 5% $NaHCO_3$ and the organic phase is dried over $Na_2SO_4$, filtered and evaporated down. The yellowish oil is quickly purified through a pad of Floresil eluting with DCM/MeOH 8/2.
HRMS (ESI): 780.2338 ($C_{40} H_{42}$ Br $N_7 O_3$ S, M+1; calc. 780.2326)
1H-NMR (400 MHz), δ (ppm, DMSO-d6): 8.42 (dd, J=4.51, 1.59 Hz, 1H), 8.08 (d, J=7.07 Hz), 7.62 (s, 1H), 7.53-7.20 (m, 9H), 7.50-7.46 (m, 2H) 7.17 (m, 2H), 7.11 (dd, J=8.17, 1.46 Hz, 1H), 6.95 (dd, J= 8.17, 4.51 Hz, 1H), 5.07 (m, 1H), 4.69 (t, J=6.58, 2H), 3.82 (s, 3H), 3.81-3.77 (m, 2H), 2.80-2.05 (m, 8H), 2.29 (s, 3H), 2.12 (s, 3H).

**Step 7. (2*E*)-2-(5-bromo-1-methyl-1H-indol-3-yl)-N-[(1S)-2-(4-methylpiperazin-1-yl)-1-phenylethyl]-3-(1H-pyrazolo[3,4-b]pyridin-3-yl)acrylamide [(R2= 5-bromo-1-methyl-1*H*-indol-3-yl,methyl-1*H*-indol-3-yl, R1 = (1*S*)-2-(4-methylpiperazin-1-yl)-1-phenylethyl] Cpd. 10**

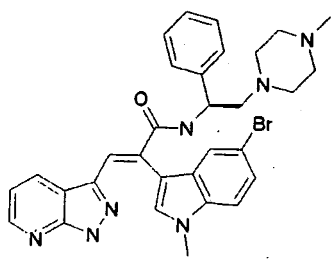

The solid obtained is then dissolved in about 10 ml of dry THF, cooled to 0°C and tBuOK (0.4 mmol) is added portionwise. The reaction is kept cool for 1 hour and then at room temperature until the cleavage reaction is complete.

After a work up with ethyl acetate, water and $NaH_2PO_4$ 20 %, the crude is purified over Floresil (DCM/ MeOH 9/1 in the presence of $NH_3$ 1 %) .
HRMS (ESI) : 598.1897 ($C_{31} H_{32}$ Br $N_7$ O, M+1; calc 598.1924)
$^1$H- NMR (400 MHz), δ (ppm, DMSO- d6) : 8.37 (dd, J= 4.32, 1.22 Hz, 1H), 8.10 (d, J = 7.07 Hz, 1H), 7.64 (s, 1H), 7.56

(s, 1H), 7.49 (d, J= 9.39 Hz, 1H), 7.40- 7.19 (m, 7H), 7.97 (d, J=7.93 Hz, 1H) 6.85 (dd, J = 8.17, 4.51 Hz, 1H), 5.05 (m, 1H), 3.80. (s, 3H) 2.67 (m, 2H) 2.46 (m, 8H), 2.12 (s, 3H) .

Operating in an analogues way, the following compounds were prepared:

**(2*E*)- *N*- [(1*S*)- 2- amino- 1- benzylethyl]- 2- (5- bromo- 1- methyl- 1*H*- indol- 3- yl)- 3- (1*H*- pyrazolo [3, 4- *b*] pyridin- 3- yl) acrylamide [R2= 5- bromo- 1- methyl- 1*H*- indol- 3- yl, R'1 = benzyl, m =1, R3= R4= H] Cpd. 11**

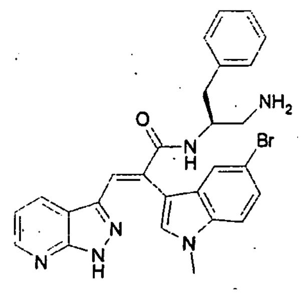

HRMS (ESI) : 529.1342 ($C_{27}H_{25}BrN_6O$, M+1; calc 529.1346)

$^1$H- NMR (400 MHz), δ (ppm, DMSO- d6) : 8.36 (dd, J= 4.51, 1.59 Hz, 1H), 7.64 (d, J= 8.29 Hz, 1H), 7.48 (s, 1H), 7.44 (d, J=8.66, 1H), 7.34- 7.19 (m, 7H), 7.12 (d, J=1.83 Hz, 1H), 7.98 (dd, J=8.17, 1.59 Hz, 1H), 6.83 (dd, J=8.17, 4.51 Hz, 1H), 4.12 (m, 1H), 3.77 (s, 3H), 2.82 (m, 2H), 2.67 (m, 2H) .

**(2*E*)-2-(5-bromo-1-methyl-1*H*-indol-3-yl)-3-(1*H*-pyrazolo[3,4-*b*]pyridin-3-yl)acrylamide [R2= 5-bromo-1-methyl-1*H*-indol-3-yl, R1 = H]**

**Cpd. 12**

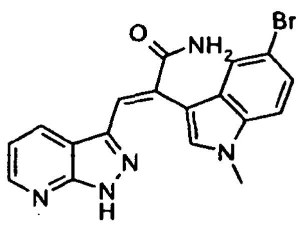

MS (ESI+) for $C_{18}H_{14}BrN_5O$ m/z = 397 ($M+H^+$)

$^1$H- NMR (400 MHz), δ (ppm, DMSO- d6) : 13.77 (s, 1H), 8.36 (dd, J=4.51, 4.39 Hz, 1H), 7.65 (s, 1H), 7.52 (s, 1H), 7.44 (d, J= 8.66 Hz, 1H), 7.36- 7.27 (m, 2H), 7.21 (dd, J= 8.66, 1.89 Hz, 1H), 7.17 (d, J=1.71 Hz, 1H), 7.03 (bd, J=7.68 Hz, 1H), 6.86 (dd, J = 8.17, 4.51 Hz, 1H), 3.78 (s, 3H) .

**(2*E*)-2-(5-bromo-1*H*-indol-3-yl)-*N*-[(1*S*)-2-(4-methylpiperazin-1-yl)-1-phenylethyl]-3-(1*H*-pyrazolo[3,4-*b*]pyridin-3-yl) acrylamide [R2= 5-bromo-1*H*-indol-3-yl, R1 = (1*S*)-2-(4-methylpiperazin-1-yl)-1-phenylethyl]**

**Cpd. 13**

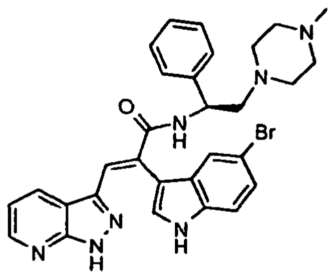

HRMS (ESI) : 584.1765 ($C_{30}$ $H_{30}$ Br $N_7$ O, M+1; calc 584.1768)
$^1$H- NMR (400 MHz), δ (ppm, DMSO- d6) : 13.80 (bs, 1H), 11.59 (d, J=2.19, 1H), 8.34 (dd, J=4.51, 1.59 Hz, 1H) 8.02 (d, J=6.95 Hz, 1H), 7.63 (s, 1H), 7.56 (d, J=2.56 Hz, 1 H), 7.39 (d, J= 8.54 Hz, 1H), 7.37- 7.30 (m, 5H), 7.23 (d, J=1.95 Hz, 1H), 7.17 (dd, J= 8.54, 1.95 Hz, 1H), 6.89 (dd, J=8.30, 1.59 Hz, 1H), 6.79 (dd, J=8.17, 4.39 Hz, 1H), 5.02 (m, 1H), 2.63 (dd, J=12.86, 9.57 Hz, 1H), 2.44 (dd, J=12.74, 5.30 Hz, 1 H), 2.41- 2.37 (m, 8H), 2.09 (bs, 3H) .

## Claims

**1.** A compound of formula (I):

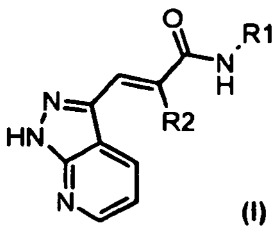

(I)

wherein:

**R1** is hydrogen or an optionally substituted group selected from straight or branched $C_1$-$C_6$ alkyl, $C_3$-$C_6$ cycloalkyl, $C_3$-$C_6$ cycloalkyl-alkyl, heterocycloalkyl, heterocycloalkyl-alkyl, aryl, arylalkyl, heteroaryl, heteroarylalkyl, aminoalkyl, hydroxyalkyl, alkoxyalkyl and a group of formula (II):

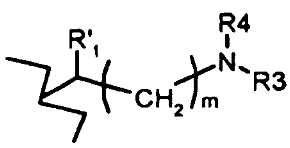

(II) ;

**R2** is hydrogen or optionally substituted aryl or heteroaryl;
**R'$_1$** is hydrogen or an optionally substituted group selected from straight or branched $C_1$-$C_6$ alkyl, $C_3$-$C_6$ cycloalkyl, $C_3$-$C_6$ cycloalkyl-alkyl, heterocycloalkyl, heterocycloalkyl-alkyl, aryl, arylalkyl, heteroaryl and heteroarylalkyl;
**m** is 0, 1 or 2;
**R3** and **R4** are, each independently, hydrogen or an optionally substituted group selected from straight or branched $C_1$-$C_6$ alkyl and $C_3$-$C_6$ cycloalkyl, or **R3** and **R4,** taken together with the nitrogen atom to which they are bonded, may form an optionally substituted heterocycloalkyl group, optionally containing an additional heteroatom selected from N, O and S;

or isomers, tautomers, solvates, hydrates or pharmaceutically acceptable salts thereof.

2. A compound of formula (I) as defined in claim 1 wherein **R1** is hydrogen or an optionally substituted group selected from aryl, arylalkyl, heteroaryl, heteroarylalkyl, aminoalkyl, hydroxyalkyl, alkoxyalkyl and a group of formula (II) as defined in claim 1;
**R2** is hydrogen or optionally substituted heteroaryl.

3. A compound or a pharmaceutically acceptable salt thereof which is selected from the group consisting of:

(2*E*)- 3- (1*H*- pyrazolo [3, 4- *b*] pyridin- 3- yl) acrylamide;
(2*E*)- N- methy- 3- (1H- pyrazolo [3, 4- b] pyridin- 3- yl) acrylamide;
(2*E*)- N- (2- morpholin- 4- ylethyl)- 3- (1H- pyrazolo [3, 4- b] pyridin- 3- yl) acrylamide;
(2*E*)- N- (2- methoxyethyl)- 3- (1H- pyrazolo [3, 4- b] pyridin- 3- yl) acrylamide;
(2*E*)- N- (2- pyrrolidin- 4- ylethyl)- 3- (1H- pyrazolo [3, 4- b] pyridin- 3- yl) acrylamide;
(2*E*)- N- benzyl- 3- (1H- pyrazolo [3, 4- b] pyridin- 3- yl) acrylamide;
(2*E*)- N- (2- chlorobenzyl)- 3- (1H- pyrazolo [3, 4- b] pyridin- 3- yl) acrylamide;
(2*E*)- N- (3- chlorobenzyl)- 3- (1H- pyrazolo [3, 4- b] pyridin- 3- yl) acrylamide;
(2*E*)- N- (4- chlorobenzyl)- 3- (1H- pyrazolo [3, 4- b] pyridin- 3- yl) acrylamide;
(2*E*)- N- (3, 4- dichlorobenzyl)- 3- (1H- pyrazolo [3, 4- b] pyridin- 3- yl) acrylamide;
(2*E*)- N- (2- fluorobenzyl)- 3- (1H- pyrazolo [3, 4- b] pyridin- 3- yl) acrylamide;
(2*E*)- N- (3- fluorobenzyl)- 3- (1H- pyrazolo [3, 4- b] pyridin- 3- yl) acrylamide;
(2*E*)- N- (4- fluorobenzyl)- 3- (1H- pyrazolo [3, 4- b] pyridin- 3- yl) acrylamide;
(2*E*)- N- (3, 4- difluorobenzyl)- 3- (1H- pyrazolo [3, 4- b] pyridin- 3- yl) acrylamide;
(2*E*)- N- (2, 4- difluorobenzyl)- 3- (1H- pyrazolo [3, 4- b] pyridin- 3- yl) acrylamide;
(2*E*)- N- (2, 5- difluorobenzyl)- 3- (1H- pyrazolo [3, 4- b] pyridin- 3- yl) acrylamide;
(2*E*)- N- (2, 6- difluorobenzyl)- 3- (1H- pyrazolo [3, 4- b] pyridin- 3- yl) acrylamide;
(2*E*)- N- (2- methoxybenzyl)- 3- (1H- pyrazolo [3, 4- b] pyridin- 3- yl) acrylamide;
(2*E*)- N- (3- methoxybenzyl)- 3- (1H- pyrazolo [3, 4- b] pyridin- 3- yl) acrylamide;
(2*E*)- N- (4- methoxybenzyl)- 3- (1H- pyrazolo [3, 4- b] pyridin- 3- yl) acrylamide;
(2*E*)- N- (4- ethoxybenzyl)- 3- (1H- pyrazolo [3, 4- b] pyridin- 3- yl) acrylamide;
(2*E*)- N- (4- methoxybenzyl)- 3- (1H- pyrazolo [3, 4- b] pyridin- 3- yl) acrylamide;
*(E)* - N- ((S)- 2- amino- 1- benzyl- ethyl)- 3- (1*H*- pyrazolo [3, 4- *b*] pyridin- 3- yl) acrylamide;
(2E)- *N*- [(2*S*)- 2- amino- 3- phenylpropyl]- 3- (1*H*- pyrazolo [3, 4- *b*] pyridin- 3- yl) acrylamide;
(2*E*)- *N*[(1*S*)- 2- amino- 1- (1*H*- indol- 3- ylmethyl) ethyl]- 3- (1*H*- pyrazolo [3, 4- *b*] pyridin- 3- yl) acrylamide;
(2*E*)- *N*- [(1*S*)- 2- amino- 1- (1- naphthylmethyl) ethyl]- 3- (1*H*- pyrazolo [3, 4- b] pyridin- 3- yl) acrylamide;
(2*E*)- *N*- [(1*S*)- 2- amino- 1- (2- naphthylmethyl) ethyl]- 3- (1*H*- pyrazolo [3, 4- *b*] pyridin- 3- yl) acrylamide;
(2*E*)- *N*- [(1*S*)- 1- phenyl- 2- pyrrolidin- 1- ylethyl]- 3- (1*H*- pyrazolo [3, 4- *b*] pyridin- 3- yl) acrylamide;
(2*E*)- *N*- [(1*S*)- 2- amino- 1- phenylethyl]- 3- (1*H*- pyrazolo [3, 4- *b*] pyridin- 3- yl) acrylamide
(2*E*)- N- [(1S)- 3- amino- 1- benzylpropyl]- 3- (1H- pyrazolo [3, 4- b] pyridin- 3- yl) acrylamide;
(2*E*)- N- [(1S)- 2- amino- 1- (thien- 2- ylmethyl) ethyl]- 3- (1H- pyrazolo [3, 4- b] pyridin- 3- yl) acrylamide;
(2*E)* - N- [(1S)- 2- morpholin- 4- yl- 1- phenylethyl]- 3- (1H- pyrazolo [3, 4- b] pyridin- 3- yl) acrylamide;
Nα- [(2*E*)- 3- (1*H*- pyrazolo [3, 4- *b*] pyridin- 3- yl) prop- 2- enoyl]- L- phenylalaninamide;
(2*E*)- *N*- [(1*S*)- 2- (4- methylpiperazin- 1- yl)- 1- phenylethyl]- 3- (1*H*- pyrazolo [3, 4- *b*] pyridin- 3- yl) acrylamide;
(2*E*)- 2- (5- bromo- 1- methyl- 1H- indol- 3- yl)- N- [(1S)- 2- (4- methylpiperazin- 1- yl)- 1- phenylethyl]- 3- (1H- pyrazolo [3, 4- b] pyridin- 3- yl) acrylamide;
(2*E*)- N- [(1S)- 2- amino- 1- benzylethyl]- 2- (5- bromo- 1- methyl- 1H- indol- 3- yl)- 3- (1H- pyrazolo [3, 4- b] pyridin- 3- yl) acrylamide;
(2*E*)- 2- (5- bromo- 1- methyl- 1H- indol- 3- yl)- 3- (1H- pyrazolo [3, 4- b] pyridin- 3- yl) acrylamide;
(2*E*)- 2- (5- bromo- 1*H*- indol- 3- yl)- *N*- [(1*S*)- 2- (4- methylpiperazin- 1- yl)- 1- phenylethyl]- 3- (1*H*- pyrazolo [3, 4- *b*] pyridin- 3- yl) acrylamide;
(2*E*)- 2- (1, 5- dimethyl- 1*H*- indol- 3- yl)- *N*- [(1*S*)- 2- (4- methylpiperazin- 1- yl)- 1- phenylethyl]- 3- (1*H*- pyrazolo [3, 4- *b*] pyridin- 3- yl) acrylamide;
(2*E*)- 2- (5- ethyl- 1- methyl- 1*H*- indol- 3- yl)- *N*- [(1*S*)- 2- (4- methylpiperazin- 1- yl)- 1- phenylethyl]- 3- (1*H*- pyrazolo [3, 4- *b*] pyridin- 3- yl) acrylamide;
(2*E*)- *N*- benzyl- 2- (5- bromo- 1- methyl- 1*H*- indol- 3- yl)- 3- (1*H*- pyrazolo [3, 4- *b*] pyridin- 3- yl) acrylamide;
(2*E*)- 2- (5- bromo- 1- methyl- 1*H*- indol- 3- yl)- *N*- [2- (dimethylamino) ethyl]- 3- (1*H*- pyrazolo [3, 4- *b*] pyridin- 3- yl) acrylamide;
(2*E*)- 2- (5- bromo- 1- methyl- 1*H*- indol- 3- yl)- *N*- (2- morpholin- 4- ylethyl)- 3- (1*H*- pyrazolo [3, 4- *b*] pyridin- 3- yl) acrylamide;
(2*E*)- 2- phenyl- 3- (1*H*- pyrazolo [3, 4- *b*] pyridin- 3- yl) acrylamide;

(2*E*)- 2- (3- bromophenyl)- 3- (1*H*- pyrazolo [3, 4- *b*] pyridin- 3- yl) acrylamide;
(2*E*)- 3- (1*H*- pyrazolo [3, 4- *b*] pyridin- 3- yl)- 2- [3- (trifluoromethyl) phenyl] acrylamide;
(2*E*)- 2- (3- bromophenyl)- *N*- [(1*S*)- 2- (4- methylpiperazin- 1- yl)- 1- phenylethyl]- 3- (1*H*- pyrazolo [3, 4- *b*] pyridin- 3- yl) acrylamide;
(2*E*)- *N*- [(1*S*)- 2- (4- methylpiperazin- 1- yl)- 1- phenylethyl]- 3- (1*H*- pyrazolo [3, 4- *b*] pyridin- 3- yl)- 2- [3- (trifluoromethyl) phenyl] acrylamide;
(2*E*)- *N*- [(1*S*)- 2- (4- methylpiperazin- 1- yl)- 1- phenylethyl]- 2- phenyl- 3- (1*H*- pyrazolo [3, 4- *b*] pyridin- 3- yl) acrylamide and
(2*E*)- 2- (3- chlorophenyl)- *N*- [(1*S*)- 2- (4- methylpiperazin- 1- yl)- 1- phenylethyl]- 3- (1*H*- pyrazolo [3, 4- *b*] pyridin- 3- yl) acrylamide.

**4.** A process for preparing a compound of formula (I) as defined in claim 1, **characterized in that** the process comprises:

e) deprotecting a compound of formula (VIII):

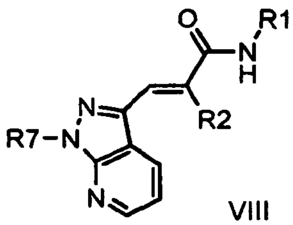

VIII

wherein **R1** and **R2** are as defined in claim 1 and **R7** is benzyl, 4-methoxybenzyl, 2,4-dimethoxybenzyl or β-tosyl ethyl; optionally separating the resulting compound into the single isomers; converting the resulting compound of formula (I) into a different compound of formula (I) and/or into a pharmaceutically acceptable salt if desired.

**5.** An in-vitro method for inhibiting the activity of one or more isoforms of the serin/threonine kinase AKT protein which comprises contacting the said protein with an effective amount of a compound as defined in claim 1.

**6.** A pharmaceutical composition comprising a therapeutically effective amount of a compound of formula (I) or a pharmaceutically acceptable salt thereof, as defined in claim 1, and at least one pharmaceutically acceptable excipient, carrier and/or diluent.

**7.** A pharmaceutical composition according to claim 6 further comprising one or more chemotherapeutic agents.

**8.** A product or kit comprising a compound of formula (I) or a pharmaceutically acceptable salt thereof, as defined in claim 1, or pharmaceutical compositions thereof as defined in claim 6 and one or more chemotherapeutic agents, as a combined preparation for simultaneous, separate or sequential use in anticancer therapy.

**9.** A compound of formula (I) or a pharmaceutically acceptable salt thereof, as defined in claim 1, for use as a medicament.

**10.** Use of a compound of formula (I) or a pharmaceutically acceptable salt thereof, as defined in claim 1, in the manufacture of a medicament with antitumor activity.

**Patentansprüche**

**1.** Verbindung mit der Formel (I) :

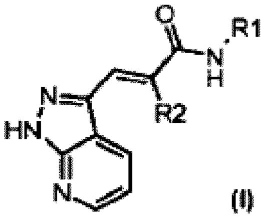

(I)

wobei:

R1 Wasserstoff oder eine wahlweise substituierte Gruppe ist, ausgewählt aus geradkettigem oder verzweigtem $C_1$- $C_6$- Alkyl, $C_3$- $C_6$- Cycloalkyl, $C_3$- $C_6$- Cycloalkyl- alkyl, Heterocycloalkyl, Heterocycloalkylalkyl, Aryl, Arylalkyl, Heteroaryl, Heteroarylalkyl, Aminoalkyl, Hydroxyalkyl, Alkoxyalkyl und einer Gruppe mit der Formel (II) :

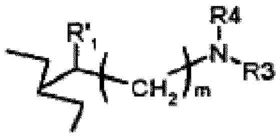

(II) ;

R2 Wasserstoff oder wahlweise substituiertes Aryl oder Heteroaryl ist;
R'$_1$ Wasserstoff oder eine wahlweise substituierte Gruppe ist, ausgewählt aus geradkettigem oder verzweigtem $C_1$- $C_6$- Alkyl, $C_3$- $C_6$- Cycloalkyl, $C_3$- $C_6$- Cycloalkyl- alkyl, Heterocycloalkyl, Heterocycloalkylalkyl, Aryl, Arylalkyl, Heteroaryl und Heteroarylalkyl;
m 0, 1 oder 2 ist;
R3 und R4 jeweils unabhängig Wasserstoff oder eine wahlweise substituierte Gruppe sind, ausgewählt aus geradkettigem oder verzweigtem $C_1$- $C_6$- Alkyl und $C_3$- $C_6$- Cycloalkyl, oder R3 und R4 zusammen mit dem Stickstoffatom, an das sie gebunden sind, eine wahlweise substituierte Heterocycloalkylgruppe bilden können, die wahlweise ein zusätzliches Heteroatom, ausgewählt aus N, 0 und S, enthält;

oder Isomere, Tautomere, Solvate, Hydrate oder pharmazeutisch akzeptable Salze davon.

**2.** Verbindung mit der Formel (I), wie in Anspruch 1 definiert, wobei R1 Wasserstoff oder eine wahlweise substituierte Gruppe ist, ausgewählt aus Aryl, Arylalkyl, Heteroaryl, Heteroarylalkyl, Aminoalkyl, Hydroxyalkyl, Alkoxyalkyl und einer Gruppe mit der Formel (II), wie in Anspruch 1 definiert;
R2 Wasserstoff oder wahlweise substituiertes Heteroaryl ist.

**3.** Verbindung oder ein pharmazeutisch akzeptables Salz davon, die aus der Gruppe ausgewählt ist, welche besteht aus:

(2E)- 3- (1H- Pyrazolo [3, 4- b] pyridin- 3- yl) acrylamid;
(2E)- N- Methyl- 3- (1H- pyrazolo [3, 4- b] pyridin- 3- yl) acrylamid;
(2E)- N- (2- Morpholin- 4- ylethyl)- 3- (1H- pyrazolo [3, 4- b] pyridin- 3- yl) acrylamid;
(2E)- N- (2- Methoxyethyl)- 3- (1H- pyrazolo [3, 4- b] pyridin- 3- yl) acrylamid;
(2E)- N- (2- Pyrrolidin- 4- ylethyl)- 3- (1H- pyrazolo [3, 4- b] pyridin- 3- yl) acrylamid;
(2E)- N- Benzyl- 3- (1H- pyrazolo [3, 4- b] pyridin- 3- yl) acrylamid;
(2E)- N- (2- Chlorbenzyl)- 3- (1H- pyrazolo [3, 4- b] pyridin- 3- yl) acrylamid;
(2E)- N- (3- Chlorbenzyl)- 3- (1H- pyrazolo [3, 4- b] pyridin- 3- yl) acrylamid;
(2E)- N- (4- Chlorbenzyl)- 3- (1H- pyrazolo [3, 4- b] pyridin- 3- yl) acrylamid;
(2E)- N- (3, 4- Dichlorbenzyl)- 3- (1H- pyrazolo [3, 4- b] pyridin- 3- yl) acrylamid;
(2E)- N- (2- Fluorbenzyl)- 3- (1H- pyrazolo [3, 4- b] pyridin- 3- yl) acrylamid;
(2E)- N- (3- Fluorbenzyl)- 3- (1H- pyrazolo [3, 4- b] pyridin- 3- yl) acrylamid;
(2E)- N- (4- Fluorbenzyl)- 3- (1H- pyrazolo [3, 4- b] pyridin- 3- yl) acrylamid;

(2E)- N- (3, 4- Difluorbenzyl)- 3- (1H- pyrazolo [3, 4- b] pyridin- 3- yl) acrylamid;
(2E)- N- (2, 4- Difluorbenzyl)- 3- (1H- pyrazolo [3, 4- b] pyridin- 3- yl) acrylamid;
(2E)- N- (2, 5- Difluorbenzyl)- 3- (1H- pyrazolo [3, 4- b] pyridin- 3- yl) acrylamid;
(2E)- N- (2, 6- Difluorbenzyl)- 3- (1H- pyrazolo [3, 4- b] pyridin- 3- yl) acrylamid;
(2E)- N- (2- Methoxybenzyl)- 3- (1H- pyrazolo [3, 4- b] pyridin- 3- yl) acrylamid;
(2E)- N- (3- Methoxybenzyl)- 3- (1H- pyrazolo [3, 4- b] pyridin- 3- yl) acrylamid;
(2E)- N- (4- Methoxybenzyl)- 3- (1H- pyrazolo [3, 4- b] pyridin- 3- yl) acrylamid;
(2E)- N- (4- Ethoxybenzyl)- 3- (1H- pyrazolo [3, 4- b] pyridin- 3- yl) acrylamid;
(2E)- N- (4- Methoxybenzyl)- 3- (1H- pyrazolo [3, 4- b] pyridin- 3- yl) acrylamid;
(E)- N- ((S)- 2- Amino- 1- benzyl- ethyl)- 3- (1H- pyrazolo [3, 4- b] pyridin- 3- yl) acrylamid;
(2E)- N- [(2S)- 2- Amino- 3- phenylpropyl]- 3- (1H- pyrazolo [3, 4- b] pyridin- 3- yl) acrylamid;
(2E)- N- [(1S)- 2- Amino- 1- (1H- indol- 3- ylmethyl) ethyl]- 3- (1H- pyrazolo [3, 4- b] pyridin- 3- yl) acrylamid;
(2E)- N- [(1S)- 2- Amino- 1- (1H- naphthylmethyl) ethyl]- 3- (1H- pyrazolo [3, 4- b] pyridin- 3- yl) acrylamid;
(2E)- N- [(1S)- 2- Amino- 1- (2- naphthylmethyl) ethyl]- 3- (1H- pyrazolo [3, 4- b] pyridin- 3- yl) acrylamid;
(2E)- N- [(1S)- 1- Phenyl- 2- pyrrolidin- 1- ylethyl]- 3- (1H- pyrazolo [3, 4- b] pyridin- 3- yl) acrylamid;
(2E)- N- [(1S)- 2- Amino- 1- phenylethyl]- 3- (1H- pyrazolo [3, 4- b] pyridin- 3- yl) acrylamid;
(2E)- N- [(1S)- 3- Amino- 1- benzylpropyl]- 3- (1H- pyrazolo [3, 4- b] pyridin- 3- yl) acrylamid;
(2E)- N- [(1S)- 2- Amino- 1- (thien- 2- ylmethyl) ethyl]- 3- (1H- pyrazolo [3, 4- b] pyridin- 3- yl) acrylamid;
(2E)- N- [(1S)- 2- Morpholin- 4- yl- 1- phenylethyl]- 3- (1H- pyrazolo [3, 4- b] pyridin- 3- yl) acrylamid;
Nα- [(2E)- 3- (1H- Pyrazolo [3, 4- b] pyridin- 3- yl) prop- 2- enoyl]- L- phenylalaninamid;
(2E)- N- [(1S)- 2- (4- Methylpiperazin- 1- yl)- 1- phenylethyl]- 3- (1H- pyrazolo [3, 4- b] pyridin- 3- yl) acrylamid;
(2E)- 2- (5- Brom- 1- methyl- 1H- indol- 3- yl)- N- [(1S)- 2- (4- methylpiperazin- 1- yl)- 1- phenylethyl]- 3- (1H- pyrazolo [3, 4- b] pyridin- 3- yl) acrylamid;
(2E)- N- [(1S)- 2- Amino- 1- benzylethyl]- 2- (5- brom- 1- methyl- 1H- indol- 3- yl)- 3- (1H- pyrazolo [3, 4- b] pyridin- 3- yl) acrylamid;
(2E)- 2- (5- Brom- 1- methyl- 1H- indol- 3- yl)- 3- (1H- pyrazolo [3, 4- b] pyridin- 3- yl) acrylamid;
(2E)- 2- (5- Brom- 1H- indol- 3- yl)- N- [(1S)- 2- (4- methylpiperazin- 1- yl)- 1- phenylethyl]- 3- (1H- pyrazolo [3, 4- b] pyridin- 3- yl) acrylamid;
(2E)- 2- (1, 5- Dimethyl- 1H- indol- 3- yl)- N- [(1S)- 2- (4- methylpiperazin- 1- yl)- 1- phenylethyl]- 3- (1H- pyrazolo [3, 4- b] pyridin- 3- yl) acrylamid;
(2E)- 2- (5- Ethyl- 1- methyl- 1H- indol- 3- yl)- N- [(1S)- 2- (4- methylpiperazin- 1- yl)- 1- phenylethyl]- 3- (1H- pyrazolo [3, 4- b] pyridin- 3- yl) acrylamid;
(2E)- N- Benzyl- 2- (5- brom- 1- methyl- 1H- indol- 3- yl)- 3- (1H- pyrazolo [3, 4- b] pyridin- 3- yl) acrylamid;
(2E)- 2- (5- Brom- 1- methyl- 1H- indol- 3- yl)- N- [2- (dimethylamino) ethyl]- 3- (1H- pyrazolo [3, 4- b] pyridin- 3- yl) acrylamid;
(2E)- 2- (5- Brom- 1- methyl- 1H- indol- 3- yl)- N- (2- morpholin- 4- ylethyl)- 3- (1H- pyrazolo [3, 4- b] pyridin- 3- yl) acrylamid;
(2E)- 2- Phenyl- 3- (1H- pyrazolo [3, 4- b] pyridin- 3- yl) acrylamid;
(2E)- 2- (3- Bromphenyl)- 3- (1H- pyrazolo [3, 4- b] pyridin- 3- yl) acrylamid;
(2E)- 3- (1H- Pyrazolo [3, 4- b] pyridin- 3- yl)- 2- [3- (trifluormethyl) phenyl] acrylamid;
(2E)- 2- (3- Bromphenyl)- N- [(1S)- 2- (4- methylpiperazin- 1- yl)- 1- phenylethyl]- 3- (1H- pyrazolo [3, 4- b] pyridin- 3- yl) acrylamid;
(2E)- N- [(1S)- 2- (4- Methylpiperazin- 1- yl)- 1- phenylethyl]- 3- (1H- pyrazolo [3, 4- b] pyridin- 3- yl)- 2- [3- (trifluormethyl) phenyl] acrylamid;
(2E)- N- [(1S)- 2- (4- Methylpiperazin- 1- yl)- 1- phenylethyl]- 2- phenyl- 3- (1H- pyrazolo [3, 4- b] pyridin- 3- yl) acrylamid und
(2E)- 2- (3- Chlorphenyl)- N- [(1S)- 2- (4- methylpiperazin- 1- yl)- 1- phenylethyl]- 3- (1H- pyrazolo [3, 4- b] pyridin- 3- yl) acrylamid.

**4.** Verfahren zum Herstellen einer Verbindung mit der Formel (I), wie in Anspruch 1 definiert, **dadurch gekennzeichnet, dass** das Verfahren umfasst:

e) Entschützen einer Verbindung mit der Formel (VIII):

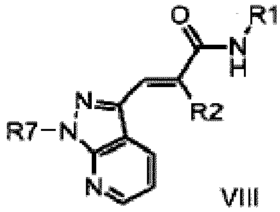

VIII

wobei R1 und R2 sind, wie in Anspruch 1 definiert, und R7 Benzyl, 4-Methoxybenzyl, 2,4-Dimethoxybenzyl oder β-Tosylethyl ist; wahlweise Trennen der entstehenden Verbindung in die einzelnen Isomere; Umwandeln der entstehenden Verbindung mit der Formel (I) in eine unterschiedliche Verbindung mit der Formel (I) und/ oder in ein pharmazeutisch akzeptables Salz, falls gewünscht.

5. In- vitro- Methode zum Hemmen der Aktivität einer oder mehrerer Isoformen des Serin/ Threonin- Kinase- AKT-Proteins, welche das Inkontaktbringen des Proteins mit einer wirksamen Menge einer Verbindung, wie in Anspruch 1 definiert, umfasst.

6. Pharmazeutische Zusammensetzung, umfassend eine therapeutisch wirksame Menge einer Verbindung mit der Formel (I) oder eines pharmazeutisch akzeptablen Salzes davon, wie in Anspruch 1 definiert, und mindestens einen pharmazeutisch akzeptablen Hilfsstoff, Träger und/oder Verdünnungsmittel.

7. Pharmazeutische Zusammensetzung nach Anspruch 6, ferner umfassend ein oder mehrere chemotherapeutische Mittel.

8. Produkt oder Kit, umfassend eine Verbindung mit der Formel (I) oder ein pharmazeutisch akzeptables Salz davon, wie in Anspruch 1 definiert, oder pharmazeutische Zusammensetzungen davon, wie in Anspruch 6 definiert, und ein oder mehrere chemotherapeutische Mittel, als ein Kombinationspräparat zur gleichzeitigen, separaten oder nacheinander erfolgenden Benutzung in der Krebstherapie.

9. Verbindung mit der Formel (I) oder ein pharmazeutisch akzeptables Salz davon, wie in Anspruch 1 definiert, zur Benutzung als ein Arzneimittel.

10. Benutzung einer Verbindung mit der Formel (I) oder eines pharmazeutisch akzeptablen Salzes davon, wie in Anspruch 1 definiert, zur Herstellung eines Arzneimittels mit Antitumor-Aktivität.

## Revendications

1. Composé de formule (1) :

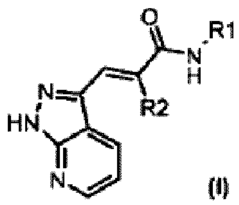

(I)

dans laquelle
R1 est un atome d'hydrogène ou un groupe facultativement substitué choisi parmi un groupe alkyle en $C_1$-$C_6$ linéaire ou ramifié, un groupe cycloalkyle en $C_3$-$C_6$, un groupe cycloalkylalkyle en $C_3$-$C_6$, un groupe hétérocycloalkyle, un groupe hétérocycloalkylalkyle, un groupe aryle, un groupe arylalkyle, un groupe hétéroaryle, un groupe hétéroarylalkyle, un groupe aminoalkyle, un groupe hydroxyalkyle, un groupe alcoxyalkyle et un groupe de formule (II) :

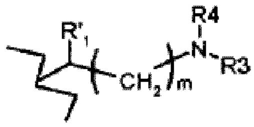

(II) ;

R2 est un atome d'hydrogène ou un groupe aryle ou hétéroaryle facultativement substitué ;
R'$_1$ est un atome d'hydrogène ou un groupe facultativement substitué choisi parmi un groupe alkyle en $C_1$-$C_6$ linéaire ou ramifié, un groupe cycloalkyle en $C_3$-$C_6$, un groupe cycloalkylalkyle en $C_3$-$C_6$, un groupe hétérocycloalkyle, un groupe hétérocycloalkylalkyle, un groupe aryle, un groupe arylalkyle, un groupe hétéroaryle et un groupe hétéroarylalkyle ;
m est 0, 1 ou 2 ;
R3 et R4 sont, chacun indépendamment, un atome d'hydrogène ou un groupe facultativement substitué choisi parmi un groupe alkyle en $C_1$-$C_6$ linéaire ou ramifié et un groupe cycloalkyle en $C_3$-$C_6$, ou R3 et R4, pris ensemble avec l'atome d'azote auquel ils sont associés, peuvent former un groupe hétérocycloalkyle facultativement substitué, contenant facultativement un hétéroatome supplémentaire choisi parmi N, 0 et S ;
ou des isomères, des tautomères, des solvates, des hydrates ou des sels pharmaceutiquement acceptables de celui-ci.

**2.** Composé de formule (I) tel que défini dans la revendication 1, dans lequel R1 est un atome d'hydrogène ou un groupe facultativement substitué choisi parmi un groupe aryle, un groupe arylalkyle, un groupe hétéroaryle, un groupe hétéroarylalkyle, un groupe aminoalkyle, un groupe hydroxyalkyle, un groupe alcoxyalkyle et un groupe de formule (II) tel que défini dans la revendication 1 ;
R2 est un atome d'hydrogène ou un groupe hétéroaryle facultativement substitué.

**3.** Composé ou sel pharmaceutiquement acceptable de celui- ci, qui est choisi dans le groupe constitué par :

le (2E)- 3- (1H- pyrazolo [3, 4- b] pyridin- 3- yl) acrylamide ;
le (2E)- N- méthyl- 3- (1H- pyrazolo [3, 4- b] pyridin- 3- yl) acrylamide ;
le (2E)- N- (2- morpholin- 4- yléthyl)- 3- (1H- pyrazolo [3, 4- b] pyridin- 3- yl) acrylamide ;
le (2E)- N- (2- méthoxyéthyl)- 3- (1H- pyrazolo [3, 4- b] pyridin- 3- yl) acrylamide ;
le (2E)- N- (2- pyrrolidin- 4- yléthyl)- 3- (1H- pyrazolo [3, 4- b] pyridin- 3- yl) acrylamide ;
le (2E)- N- benzyl- 3- (1H- pyrazolo [3, 4- b] pyridin- 3- yl) acrylamide ;
le (2E)- N- (2- chlorobenzyl)- 3- (1H- pyrazolo [3, 4- b] pyridin- 3- yl) acrylamide ;
le (2E)- N- (3- chlorobenzyl)- 3- (1H- pyrazolo [3, 4- b] pyridin- 3- yl) acrylamide ;
le (2E)- N- (4- chlorobenzyl)- 3- (1H- pyrazolo [3, 4- b] pyridin- 3- yl) acrylamide ;
le (2E)- N- (3, 4- dichlorobenzyl)- 3- (1H- pyrazolo [3, 4- b] pyridin- 3- yl) acrylamide ;
le (2E)- N- (2- fluorobenzyl)- 3- (1H- pyrazolo [3, 4- b] pyridin- 3- yl) acrylamide ;
le (2E)- N- (3- fluorobenzyl)- 3- (1H- pyrazolo [3, 4- b] pyridin- 3- yl) acrylamide ;
le (2E)- N- (4- fluorobenzyl)- 3- (1H- pyrazolo [3, 4- b] pyridin- 3- yl) acrylamide ;
le (2E)- N- (3, 4- difluorobenzyl)- 3- (1H- pyrazolo [3, 4- b] pyridin- 3- yl) acrylamide ;
le (2E)- N- (2, 4- difluorobenzyl)- 3- (1H- pyrazolo [3, 4- b] pyridin- 3- yl) acrylamide ;
le (2E)- N- (2, 5- difluorobenzyl)- 3- (1H- pyrazolo [3, 4- b] pyridin- 3- yl) acrylamide ;
le (2E)- N- (2, 6- difluorobenzyl)- 3- (1H- pyrazolo [3, 4- b] pyridin- 3- yl) acrylamide ;
le (2E)- N- (2- méthoxybenzyl)- 3- (1H- pyrazolo [3, 4- b] pyridin- 3- yl) acrylamide ;
le (2E)- N- (3- méthoxybenzyl)- 3- (1H- pyrazolo [3, 4- b] pyridin- 3- yl) acrylamide ;
le (2E)- N- (4- méthoxybenzyl)- 3- (1H- pyrazolo [3, 4- b] pyridin- 3- yl) acrylamide ;
le (2E)- N- (4- éthoxybenzyl)- 3- (1H- pyrazolo [3, 4- b] pyridin- 3- yl) acrylamide ;
le (2E)- N- (4- méthoxybenzyl)- 3- (1H- pyrazolo [3, 4- b] pyridin- 3- yl) acrylamide ;
le (E)- N- ((S)- 2- amino- 1- benzyl- éthyl)- 3- (1H- pyrazolo [3, 4- b] pyridin- 3- yl) acrylamide ;
le (2E)- N- [(2S)- 2- amino- 3- phénylpropyl]- 3- (1H- pyrazolo [3, 4- b] pyridin- 3- yl) acrylamide ;
le (2E)- N- [(1S)- 2- amino- 1- (1H- indol- 3- ylméthyl) éthyl]- 3- (1H- pyrazolo [3, 4- b] pyridin- 3- yl) acrylamide ;
le (2E)- N- [(1S)- 2- amino- 1- (1- naphtylméthyl) éthyl]- 3- (1H- pyrazolo [3, 4- b] pyridin- 3- yl) acrylamide ;
le (2E)- N- [(1S)- 2- amino- 1- (2- naphtylméthyl) éthyl]- 3- (1H- pyrazolo [3, 4- b] pyridin- 3- yl) acrylamide ;
le (2E)- N- [(1S)- 1- phényl- 2- pyrrolidin- 1- yléthyl]- 3- (1H- pyrazolo [3, 4- b] pyridin- 3- yl) acrylamide ;

le (2E)- N- [(1S)- 2- amino- 1- phényléthyl]- 3- (1H- pyrazolo [3, 4- b] pyridin- 3- yl) acrylamide ;
le (2E)- N- [(1S)- 3- amino- 1- benzylpropyl]- 3- (1H- pyrazolo [3, 4- b] pyridin- 3- yl) acrylamide ;
le (2E)- N- [(1S)- 2- amino- 1- (thién- 2- ylméthyl) éthyl]- 3- (1H- pyrazolo [3, 4- b] pyridin- 3- yl) acrylamide ;
le (2E)- N- [(1S)- 2- morpholin- 4- yl- 1- phényléthyl]- 3- (1H- pyrazolo [3, 4- b] pyridin- 3- yl) acrylamide ;
le Nα- [(2E)- 3- (1H- pyrazolo [3, 4- b] pyridin- 3- yl) prop- 2- énoyl]- L- phényl- alaninamide ;
le (2E)- N- [(1S)- 2- (4- méthylpipérazin- 1- yl)- 1- phényléthyl]- 3- (1H- pyrazolo [3, 4- b] pyridin- 3- yl) acrylamide ;
le (2E)- 2- (5- bromo- 1- méthyl- 1H- indol- 3- yl)- N- [(1S)- 2- (4- méthylpipérazin- 1- yl)- 1- phényléthyl]- 3- (1H- pyrazolo [3, 4- b] pyridin- 3- yl) acrylamide ;
le (2E)- N- [(1S)- 2- amino- 1- benzyléthyl]- 2- (5- bromo- 1- méthyl- 1H- indol- 3- yl)- 3- (1H- pyrazolo [3, 4- b] pyridin- 3- yl) acrylamide ;
le (2E)- 2- (5- bromo- 1- méthyl- 1H- indol- 3- yl)- 3- (1H- pyrazolo [3, 4- b] pyridin- 3- yl) acrylamide ;
le (2E)- 2- (5- bromo- 1H- indol- 3- yl)- N- [(1S)- 2- (4- méthylpipérazin- 1- yl)- 1- phényléthyl]- 3- (1H- pyrazolo [3, 4- b] pyridin- 3- yl) acrylamide ;
le (2E)- 2- (1, 5- diméthyl- 1H- indol- 3- yl)- N- [(1S)- 2- (4- méthylpipérazin- 1- yl)- 1- phényléthyl]- 3- (1H- pyrazolo [3, 4- b] pyridin- 3- yl) acrylamide ;
le (2E)- 2- (5- éthyl- 1- méthyl- 1H- indol- 3- yl)- N- [(1S)- 2- (4- méthylpipérazin- 1- yl)- 1- phényléthyl]- 3- (1H- pyrazolo [3, 4- b] pyridin- 3- yl) acrylamide ;
le (2E)- N- benzyl- 2- (5- bromo- 1- méthyl- 1H- indol- 3- yl)- 3- (1H- pyrazolo [3, 4- b] pyridin- 3- yl) acrylamide ;
le (2E)- 2- (5- bromo- 1- méthyl- 1H- indol- 3- yl)- N- [2- (diméthylamino) éthyl]- 3- (1H- pyrazolo [3, 4- b] pyridin- 3- yl) acrylamide ;
le (2E)- 2- (5- bromo- 1- méthyl- 1H- indol- 3- yl)- N- (2- morpholin- 4- yléthyl)- 3- (1H- pyrazolo [3, 4- b] pyridin- 3- yl) acrylamide ;
le (2E)- 2- phényl- 3- (1H- pyrazolo [3, 4- b] pyridin- 3- yl) acrylamide ;
le (2E)- 2- (3- bromophényl)- 3- (1H- pyrazolo [3, 4- b] pyridin- 3- yl) acrylamide ;
le (2E)- 3- (1H- pyrazolo [3, 4- b] pyridin- 3- yl)- 2- [3- (trifluorométhyl) phényl]- acrylamide ;
le (2E)- 2- (3- bromophényl)- N- [(1S)- 2- (4- méthylpipérazin- 1- yl)- 1- phényléthyl]- 3- (1H- pyrazolo [3, 4- b] pyridin- 3- yl) acrylamide ;
le (2E)- N- [(1S)- 2- (4- méthylpipérazin- 1- yl)- 1- phényléthyl]- 3- (1H- pyrazolo [3, 4- b] pyridin- 3- yl)- 2- [3- (trifluorométhyl) phényl] acrylamide ;
le (2E)- N- [(1S)- 2- (4- méthylpipérazin- 1- yl)- 1- phényléthyl]- 2- phényl- 3- (1H- pyrazolo [3, 4- b] pyridin- 3- yl) acrylamide ; et
le (2E)- 2- (3- chlorophényl)- N- [(1S)- 2- (4- méthylpipérazin- 1- yl)- 1- phényléthyl]- 3- (1H- pyrazolo [3, 4- b] pyridin- 3- yl) acrylamide.

**4.** Procédé de préparation d'un composé de formule (I) selon la revendication 1, **caractérisé en ce que** le procédé comprend :

e) la déprotection d'un composé de formule (VIII) :

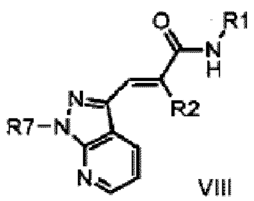

VIII

dans laquelle R1 et R2 sont tels que définis dans la revendication 1 et R7 est un groupe benzyle, un groupe 4-méthoxybenzyle, un groupe 2,4-diméthoxybenzyle ou un groupe β-tosyléthyle ; la séparation facultative du composé résultant en des isomères uniques ; la conversion du composé résultant de formule (I) en un composé différent de formule (I) et/ou en un sel pharmaceutiquement acceptable si souhaité.

**5.** Méthode in vitro d'inhibition de l'activité d'une ou plusieurs isoformes de la protéine sérine/thréonine kinase AKT qui comprend la mise en contact de ladite protéine avec une quantité efficace d'un composé tel que défini dans la revendication 1.

**6.** Composition pharmaceutique comprenant une quantité thérapeutiquement efficace d'un composé de formule (I) ou d'un sel pharmaceutiquement acceptable de celui-ci, tel que défini dans la revendication 1, et d'au moins un excipient, véhicule et/ou diluant pharmaceutiquement acceptable.

**7.** Composition pharmaceutique selon la revendication 6, comprenant en outre un ou plusieurs agents chimiothérapeutiques.

**8.** Produit ou kit comprenant un composé de formule (I) ou un sel pharmaceutiquement acceptable de celui-ci, tel que défini dans la revendication 1, ou compositions pharmaceutiques de celui-ci telles que définies dans la revendication 6 et un ou plusieurs agents chimiothérapeutiques, sous forme de préparation combinée pour une utilisation simultanée, séparée ou séquentielle dans le cadre d'un traitement anticancéreux.

**9.** Composé de formule (I) ou un sel pharmaceutiquement acceptable de celui-ci, tel que défini dans la revendication 1, pour une utilisation en tant que médicament.

**10.** Utilisation d'un composé de formule (I) ou un sel pharmaceutiquement acceptable de celui-ci, tel que défini dans la revendication 1, dans la fabrication d'un médicament ayant une activité antitumorale.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2003037862 A
- WO 2005011681 A
- WO 200198299 A
- EP 384349 A1


### Non-patent literature cited in the description


- *Current Opinion in Chemical Biology,* 1999, vol. 3, 459-465
- *Proc. Natl. Acad. Sci.,* 2001, vol. 98, 10983-10985
- *J. Cell. Sci.,* 2001, vol. 114, 2903-2910
- *Circ. Res.,* 2000, vol. 86, 15-23
- *Proc. Natl. Acad. Sci.,* 1992, vol. 89, 9267-9271
- *J. Biol. Chem.,* 1999, vol. 274, 9133-9136
- *Cell. Signal.,* 2002, vol. 14, 381-395
- *Nat. Cell. Biol.,* 1999, vol. 1, 500-506
- *J. Biol. Chem.,* 1999, vol. 274, 1865-1868
- *J. Clin. Invest.,* 1999, vol. 106, 493-499
- *Nat. Med.,* 2000, vol. 6, 1004-1010
- *Proc. Natl. Acad. Sci,* 1996, vol. 93, 3636-3641
- *Int. J. Cancer,* 1995, vol. 64, 280-285
- *Nature,* 1999, vol. 401, 33-34
- *Oncogene,* 2000, vol. 19, 2324-2330
- *Neurosci.,* 2000, vol. 20, 2875-2886
- *Proc. Natl. Acad.,* 2001, vol. 98, 7200-7205
- *Nature,* 2001, vol. 411, 355-365
- *Nat. Rev. Cancer,* 2002, vol. 2, 489-501
- **CACCHI S. et al.** *Org. Lett.,* 2003, vol. 5 (23), 4269
- *Can.J.Chem.,* 1988, vol. 66, 420-428
- *Tetrahedron Letters,* 2002, vol. 43, 2695-2697
- *Tetrahedron,* 2004, vol. 60, 901
- **SMITH, MICHAEL ; MICHAEL B. SMITH ; JERRY MARCH.** March's Advanced Organic Chemistry: reactions mechanisms and structure. John Wiley & Sons Inc, 2001
- **GREEN, THEODORA W. ; WUTS, PETER G.M.** Protective Groups in Organic Synthesis. John Wiley & Sons Inc, 1999
- **E.L.ELIEL ; S.H. WILEN ; L.N. MANDER.** Stereochemistry of Organic Compounds. Wiley-Interscience, 1994
- **KUMAR, C. C.** *Biochem. Biophys. ACTA,* 2001, vol. 1526, 257-268
- **ALESSI, D. R.** *Biochem. J.,* 1998, vol. 331, 299-308
- **C. DALVIT et al.** *J. Am. Chem. Soc.,* 2003, vol. 125 (47), 14620
- *Tetrahedron Lett.,* 2000, vol. 41, 4363
- *Can. J.Chem.,* 1988, vol. 66, 420-428
- *Synth. Comm.,* 2000, vol. 30 (16), 2897-2902
- *Org, Lett. 5,* 2003, vol. 23, 4269-4272